# FASCICULE DE BREVET EUROPEEN

(11) **EP 0 946 566 B1**
(45) Date de publication et mention de la délivrance du brevet: **05.11.2003**
(21) Numéro de dépôt: 97950235.8
(22) Date de dépôt: 05.12.1997
(51) Int. Cl.: C07D 498/22, A61K 31/47, C07D 491/22, C07D 471/14

(54) **FORMES PRODROGUES ET ANALOGUES DE LA CAMPTOTHECINE, LEUR APPLICATION COMME MEDICAMENTS**
PRODROGS UND ANALOGA VON CAMPTOTHECIN UND DEREN VERWENDUNG ALS ARZNEIMITTEL
PRO-DRUGS AND COUNTERPARTS OF CAMPTOTHECIN, THEIR APPLICATION AS MEDICINES

(30) Priorité: 20.12.1996 FR 9615775; 24.12.1996 FR 9615945
(43) Date de publication de la demande: 06.10.1999
(73) Titulaire: SOCIETE DE CONSEILS DE RECHERCHES ET D'APPLICATIONS SCIENTIFIQUES (S.C.R.A.S.), 75016 Paris (FR)
(72) Inventeur: BIGG, Dennis, F-91190 Gif sur Yvette (FR); LAVERGNE, Olivier, F-91300 Massy (FR); HARNETT, Jerry, F-91190 Gif sur Yvette (FR); ROLLAND, Alain, F-91120 Palaiseau (FR); LIBERATORE, Anne-Marie, F-78610 Auffargis (FR); LANCO, Christophe, F-92190 Meudon (FR)
(74) Mandataire: Bourgouin, André
(86) Numéro de dépôt international: FR9702217
(87) Numéro de publication internationale: WO98028304

(56) Documents cités:
- WO-A-97/00876

## Description

La camptothécine est un composé naturel qui a été isolé pour la première fois des feuilles et de l'écorce de la plante chinoise appelée *camptotheca acuminata* (voir Wall et ses collaborateurs, J. Amer. Chem. Soc. 88:3888 (1966)). La camptothécine est un composé pentacyclique constitué d'un fragment indolizino[1,2-b]quinoléine (cycles A, B, C et D) fusionné avec une α-hydroxylactone à six chaînons (cycle E). Le carbone en position 20, qui porte le groupe α-hydroxy, est asymétrique et confère à la molécule un pouvoir rotatoire. La forme naturelle de la camptothécine possède la configuration absolue "S" au carbone 20 et répond à la formule suivante :

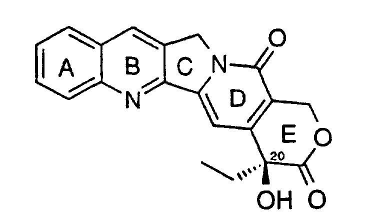

La camptothécine et ses analogues présentent une activité anti-proliférative dans plusieurs lignées cellulaires cancéreuses, comprenant les lignées cellulaires de tumeurs humaines du colon, du poumon et du sein (Suffness, M. et collaborateurs : The Alkaloids Chemistry and Pharmacology, Bross, A., ed., Vol. 25, p. 73 (Academic Press, 1985)). On suggère que l'activité anti-proliférative de la camptothécine est en relation avec son activité inhibitrice de la topoisomérase I de l'ADN.

Par ailleurs, la camptothécine et certains de ses analogues ne sont pas hydrosolubles, ce qui rend difficile leur administration par voie parentérale. Il a été préparé des dérivés hydrosolubles de la camptothécine où les cycles A et B portent des substituants salifiables (cf., par exemple, US 4,981,968, US 5,049,668, EP 540,099). Mais ces produits ont révélé une activité antitumorale amoindrie par rapport à celle des dérivés non-hydrosolubles. Il a également été préparé d'autres dérivés hydrosolubles de la camptothécines où le groupe hydroxyle de la position 20 est estérifié par un acide portant un radical salifiable, tel que par exemple la glycine (cf. les brevets US No. 4,943,579 et PCT No. WO 96/02546). Ces dérivés sont désignés par l'homme de l'art par le nom de "formes prodrogues" car ils ne sont pas biologiquement actifs en tant que tel, mais seulement après une première étape de métabolisation une fois administré au patient. Les formes prodrogues des analogues α-hydroxylactoniques de la camptothecine ont montré une bonne efficacité anti-tumorale chez l'animal et en clinique, mais accompagnée d'effets secondaires néfastes tels que l'apparition de diarrhées graves qui peuvent mettre en danger la vie du patient. Il est donc nécessaire de développer des analogues hydrosolubles de le camptothécine qui soient plus efficaces et mieux tolérés.

Par ailleurs, il a été indiqué que l'α-hydroxylactone était une exigence absolue à la fois pour l'activité *in vivo* et *in vitro* des camptothécines (Camptothecins : New Anticancer Agents, Putmesil, M., et ses collaborateurs, ed., p. 27 (CRC Press, 1995) ; Wall, M. et ses collaborateurs, Cancer Res. 55:753 (1995); Hertzberg et ses collaborateurs, J. Med. Chem. 32:715 (1982) et Crow et ses collaborateurs, J. Med. Chem. 35:4160 (1992)). Cependant, la demanderesse a découvert que les β-hydroxylactones à 7 chaînons ont une activité biologique comparable ou supérieure à celle des α-hydroxylactones (demande PCT non publiée No. FR 96/00980). La présente invention concerne de nouveaux dérivés de cette classe d'analogues de la camptothécine, dans lesquels une β-hydroxylactone à sept chaînons remplace l'α-hydroxylactone naturelle de la camptothécine. On entend par β-hydroxylactone une lactone qui comporte un atome de carbone supplémentaire entre le carbone du carboxyle et le carbone-α portant l'hydroxyle dans α-hydroxylactone.

Deux solutions ont été choisies afin d'augmenter l'hydrosolubilité des analogues de la camptothécine : la première consiste à greffer une oxazine sur le cycle A de la molécule, et la seconde à concevoir des formes prodrogues en acétylant la fonction hydroxy de la β-hydroxylactone.

Plus précisément, parmi cette nouvelle classe d'analogues de la camptothécine, les composés selon la présente invention sont soit des analogues modifiés par fixation d'un cycle oxazine sur les carbones 10 et 11, soit des formes prodrogues dans lesquels une β-hydroxylactone remplace l'α-hydroxylactone naturelle de la camptothécine. Les composés de la présente invention sont donc des β-hydroxylactones analogues de la camptothécine sur lesquelles on a greffé un cycle oxazine ou des prodrogues hydrosolubles et présentent une activité biologique puissante qui est inattendue au regard de l'état de l'art antérieur.

L'invention a plus particulièrement pour objet des composés de formule (I): 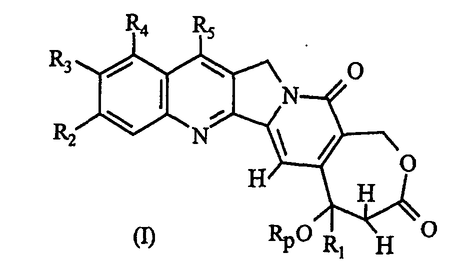 sous forme racémique, d'énantiomère ou toutes combinaisons de ces formes, dans laquelle
- R₁: représente le groupe éthyle ;
- R₂: représente H ou un halo ;
- R₃: représente H, un alkyle inférieur, halo, ou OR₆ dans lequel R₆ représente H, un alkyle inférieur ou un aryle alkyle inférieur ;
- R₄: représente H ou -(CH₂)ₘNR₆R₇ dans lequel R₆ et R₇ représentent, indépendamment, H ou un alkyle inférieur ;
- R₅: représente H, un alkyle inférieur ou -(CH₂)ₙ[N≈X] substitué ou non substitué et [N≈X] représente le groupe pipéridyle, morpholinyle, pipérazinyle ou indolyle et ledit substituant est un alkyle inférieur ;
ou R₃ et R₄ forment un cycle oxazine éventuellement substitué sur l'atome d'azote par un radical R₉ qui représente un alkyle inférieur ;
- Rp: représente H ou un groupe facilement clivable de formule -C(O)-(A₁)-NH₂ dans lequel A₁ représente (CH₂)ₘ ou un radical alkylène inférieur ramifié ;
- m: est un nombre entier compris entre 0 et 6;
- n: est 1 ou 2; et
étant entendu que lorsque Rₚ est un atome d'hydrogène, R₃ et R₄ forment ensemble un cycle oxazine éventuellement substitué,
et lorsque le terme inférieur est associé à alkyle ou alkylène, il signifie au plus 6 atomes de carbone;
ou un sel pharmaceutiquement acceptable de ce dernier.

Tel qu'il est utilisé ici, le terme inférieur en référence aux groupes alkyle, ou alkylène désigne des groupes hydrocarbonés aliphatiques saturés, linéaires, ou ramifiés, comportant de 1 à 6 carbones, comme par exemple méthyle, éthyle, propyle, isopropyle, butyle, t-butyle, vinyle, allyl, isopropényle, pentènyle, hexanyle et propènyle éthynyle.

Les composés selon la présente invention ont deux formes énantiomères possibles, c'est-à-dire sous les configurations "R" et "S". La présente invention inclut les deux formes éniantiomères et toutes combinaisons de ces formes, y compris les mélanges racémiques "RS". Dans un souci de simplicité, lorsqu'aucune configuration spécifique n'est indiquée dans les formules de structure, il faut comprendre que les deux formes énantiomères et leurs mélanges sont représentés.

Des exemples de camptothécines substituées, utilisées comme produits de départ, peuvent être trouvés dans les brevets américains Nos. 4 473 692, 4 604 463, 4 894 956, 5 162 532, 5 395 939, 5 315 007, 5 264 579, 5 258 516, 5 254 690, 5 212 317 et 5 341 745, les demandes de brevet PCT Nos. US91/08028, US94/06451, US90/05172, US92/04611, US93/10987, US91/09598, EP94/03058 et EP95/00393 et les demandes de brevets européens Nos. 325 247, 495 432, 321 122 et 540 099.

Pour les composés comportant un cycle oxazine :
- l'on traite avec une amine primaire, dans les conditions de Mannich, un composé β-hydroxylactonique de formule générale D 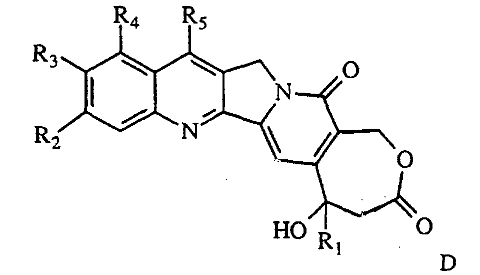
dans laquelle R₃ est un radical hydroxyle, R₄ est H, et R₁ et R₂ ont la signification indiquée ci-dessus pour obtenir un composé β-hydroxylactonique de formule générale Ia 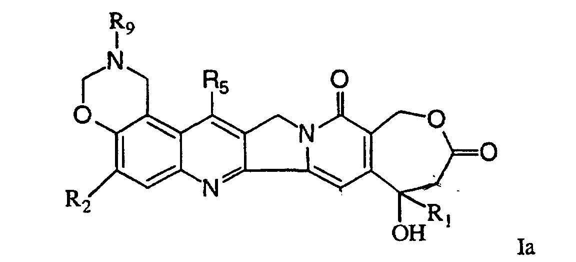 dans laquelle R₁, R₂, R₅ et R₉ ont la signification indiquée ci-dessus.

Ce procédé consiste à chauffer à une température de 30° C à 80° C durant un temps allant de 0,5 à 5 heures le produit de départ en présence d'une amine primaire telle que la benzylamine, de formaldéhyde dans un solvant acide tel que l'acide acétique ou l'acide propionique. On peut alternativement chauffer à une température de 30° C à 80° C durant un temps allant de 0,5 à 5 heures une suspension du produit de départ dans l'acide acétique par une hexahydrotriazine tri-*N*-substituée telle que la hexahydro-1,3,5-triméthyl triazine, la 1,3,5-triéthylhexahydro triazine ou la 1,3,5-tribenzyl hexahydrotriazine.
- l'on acyle éventuellement ledit composé de formule générale D ou la, de préférence avec un dérivé du radical C(O)-A₁-N-H₂ comme défini ci-dessus, pour donner le composé β-hydroxylactonique de la formule générale I avec Rₚ différent de H (forme prodrogue de l'invention).

Dans le procédé ci-dessus, les groupes R₂, R₃, R₄ et R₅ peuvent être protégés si nécessaire selon les méthodes classiques de protection (Greene, T., Protective Groups in Organic Synthesis 10-86 (John Wiley & Sons 1981)). Si au moins l'un des groupements R₂₂ ou R₂₃ est H, ou contient au moins une fonction chimiquement incompatible avec le procédé d'acylation telle que, par exemple, une amine primaire ou secondaire, il est alors nécessaire d'avoir recours à un groupe protecteur résistant aux conditions d'acylation. Un groupe protecteur communement employé pour les amines est le *tert*-butyloxycarbonyl (BOC). La réaction d'acylation est alors effectuée tel que décrit ci-dessus, puis le groupe protecteur est clivé, par exemple par un traitement à l'acide trifluoroacétique dans le cas du BOC, pour donner le composé de formule générale (I) ou (II). L'usage des groupes protecteurs est connu de l'homme de l'art (pour d'autres exemples, on peut se référer à Greene, T., Protectives Groups in Organic Synthesis, John Wiley & Sons, 1981).

Les composés de formule D sont préparés comme suit :
- l'on couple un composé de formule générale M 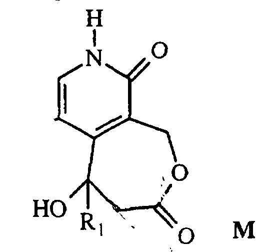
dans laquelle R₁ a la signification indiquée ci-dessus et R₂₀ représente l'hydrogène ou un atome d'halogène, avec un 2-halo-3-quinoléine-méthanol de formule générale N 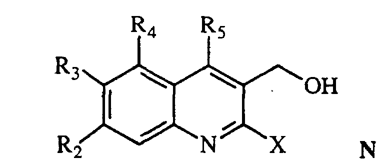 dans laquelle R₂, R₃, R₄ et R₅ ont la signification indiquée ci-dessus et X représente un atome d'halogène, pour donner le composé de formule O 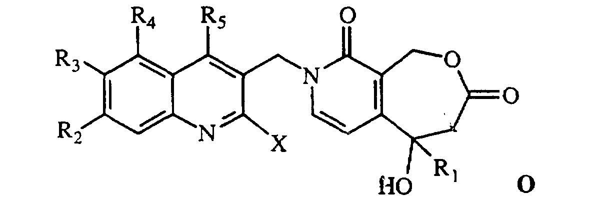
- puis l'on cyclise le composé de formule générale O pour obtenir le composé de formule générale D telle que définie ci-dessus.

Dans le procédé ci-dessus, les groupes R₁, R₂, R₃ et R₄ peuvent être protégés si nécessaire selon les méthodes classiques de protection (Greene, T., Protective Groups in Organic Synthesis 10-86 (John Wiley & Sons 1981)). La formation du composé O à partir des composés de formule générale M et N s'effectue par un traitement connu de l'homme de l'art sous le nom de réaction de Mitsunobu (se référer à Mitsunobu, O. et coll. *Synthesis*, p.1 (1981)). Il s'agit de déplacer la fonction hydroxyle du composé N par un nucléophile tel que le composé M, ou un dérivé déprotoné de ce dernier, par un traitement avec une phosphine, par exemple la triphénylphosphine, et un dérivé azodicarboxylate, par exemple l'azodicarboxylate de diéthyle, dans un solvant aprotique tel que, par exemple, le tétrahydrofurane ou le *N,N*-diméthylformamide. La cyclisation du composé O s'effectue de préférence en présence d'un catalyseur palladié (par exemple le diacétate de palladium) dans des conditions basiques (fournies par exemple par un acétate alcalin éventuellement combiné à un agent de transfert de phase tel que par exemple le bromure de tétrabutylammonium), dans un solvant aprotique tel que l'acétonitrile ou le *N,N*-diméthylformamide, à une température comprise entre 50° C et 120° C (R. Grigg et coll., *Tetrahedron* 46, page 4003 (1990)).

Les composés de formule générale M peuvent être préparés selon un procédé caractérisé en ce que
- l'on protège le carbonyle d'une pyridine de formule générale 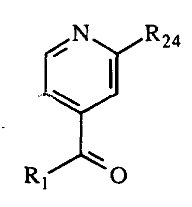
dans laquelle R₁ a la signification indiquée ci-dessus et R₂₄ représente un atome d'halogène ou un alkoxy inférieur, par une fonction acétal, pour donner le composé de formule générale F 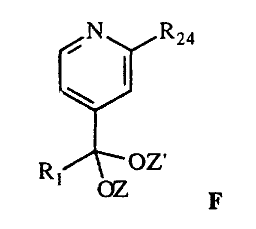 dans laquelle les groupes Z et Z' représentent, indépendamment, un alkyl inférieur ou forment ensemble une chaîne hydrocarbonée saturée de 2 à 4 carbones;
- l'on introduit dans le composé de formule générale F une fonction hydroxyméthyle pour obtenir un composé de formule générale G 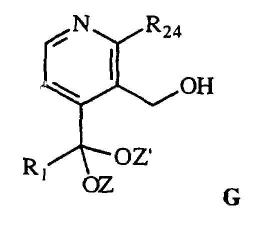
- puis l'on protège la fonction alcoolique du composé de formule générale G pour donner un composé de formule générale H 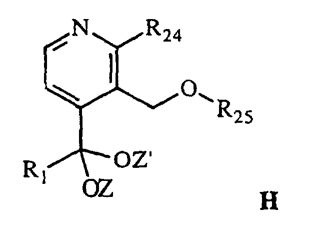
dans laquelle R₁ et Z et Z' ont la signification indiquée ci-dessus et R₂₅ représente un groupe protecteur de la fonction alcool,
- l'on déprotège l'acétal du composé de formule générale H pour donner le composé de formule générale I' 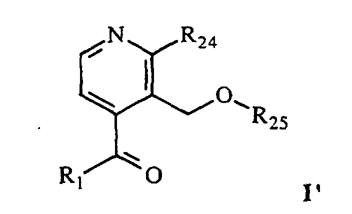
- l'on traite par un agent alkylant fonctionnalisé le composé de formule I' pour donner un β-hydroxyester de formule générale J 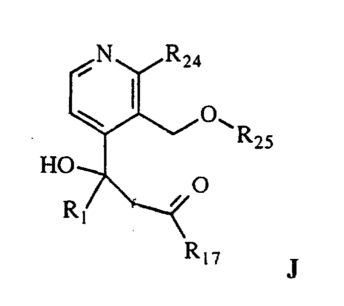
dans laquelle R₁, R₂₄ et R₂₅ ont la signification indiquée ci-dessus et R₁₇ représente OR₆ ou NHR₆ et R₆ représente H, un alkyle inférieur, cycloalkyle, cycloalkyle alkyle inférieur, alkényle inférieur, alkoxy inférieur alkyle inférieur, ou aryl ou aryle alkyle inférieur,
- l'on clive le groupe protecteur R₂₅ du composé de formule générale J, pour donner un composé de formule générale K, 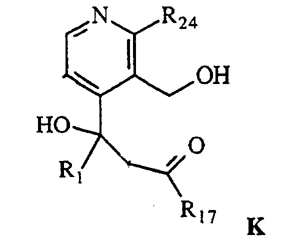
dans laquelle R₁, R₁₈, R₁₉, R₂₀ et R₂₄ ont la signification indiquée ci-dessus et R₁₇
- l'on cyclise le composé de formule générale K en composé de formule générale L 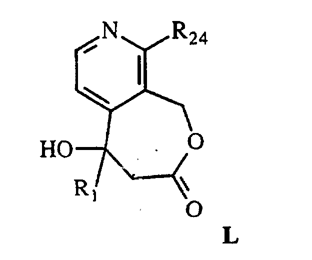 et enfin
- l'on transforme le radical R₂₄ du composé L en carbonyle, pour obtenir le composé de formule générale M

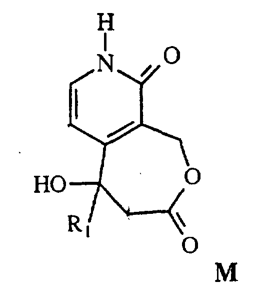

La fonction carbonyle d'une 4-acyl-2-halopyridine (obtenue par exemple selon Lamattina, J.L. *J. Hétérocyclic Chem. 20*, *p.* 553 (1983)) est protégée de préférence par une fonction acétal, de préférence acétal cyclique, selon des condition classiques connues de l'homme de l'art (Greene, T., Protective Groups in Organic Synthesis 10-86 (John Wiley & Sons 1981)). L'intermédiaire ainsi obtenu est traité par un alcoolate de sodium ou de potassium dans un solvant aprotique (par exemple l'acétonitrile), ou dans l'alcool dont est dérivé l'alcoolate, à une température comprise entre 0° C et 100° C pour donner le composé de formule générale F. Ce dernier peut être lithié en position 3 par traitement par un aryl- ou alkyl-lithium (par exemple mésityl-lithium) dans un solvant éthéré tel que le tétrahydrofurane à une température comprise entre -100° C et 0° C. A l'intermédiaire lithié ainsi obtenu, on ajoute un électrophile formylant tel que le *N,N*-diméthylformamide, et l'aldéhyde ainsi obtenu, après hydrolyse, est traité par un agent réducteur tel que le borohydrure de sodium pour donner le composé de formule générale G. La protection de la fonction alcool du composé G s'effectue selon des conditions classiques connues de l'homme de l'art, pour obtenir un composé de formule générale H. Des exemples de groupes protecteurs de la fonction alcool comprennent ceux qui forment les éthers [c'est-à-dire, méthyle, méthoxyméthyle, tétrahydropyranyl, 2-méthoxyéthoxy méthyle, benzyloxyméthyle, t-butyl, et benzyl (substitué ou non)], et les esters (c'est-à-dire formate, acétate et isobutyrate). Pour d'autres exemples de groupes protecteurs d'hydroxyl primaires, on peut se référer à Greene, T., Protectives Groups in Organic Synthesis, 10-86 (John Wiley & Sons, 1981). La déprotection du composé de formule générale H pour donner le composé de formule générale I' s'effectue dans des conditions sélectives maintenant l'intégrité du radical R₂₅, par exemple, par traitement dans des conditions acides (par exemple par l'acide trifluoroacétique). Les conditions sélectives de protection et de déprotection des groupes fonctionnels sont connues de l'homme de l'art (Greene, T., Protective Groups in Organic Synthesis 10-86 (John Wiley & Sons 1981)). Le traitement du composé I' par un agent alkylant fonctionnalisé pour donner un β-hydroxyester de formule générale J, peut être réalisée à l'aide d'un énolate de lithium ou d'un dérivé zincique d'un ester carboxylique dans un solvant aprotique anhydre, par exemple le tétrahydrofurane. Le groupe protecteur R₂₅ du composé de formule générale J, est clivé pour donner un composé de formule générale K, dans des conditions de déprotection connues de l'homme de l'art. Par exemple, lorsque R₂₅ est un groupe benzyle, on peut soumettre une solution alcoolique du composé de formule générale J additionnée d'un catalyseur palladié à une atmosphère d'hydrogène sous une pression de 0,5 à 10 Bar. La cyclisation du composé de formule générale K ainsi obtenu peut être effectuée dans des conditions acides (par exemple par traitement par l'acide trifluoroacétique, ou le gaz chlorhydrique dissout dans un solvant anhydre tel que le dichlorométhane ou le dioxane) pour donner un cycle β-hydroxylactonique à sept chaînons tel que dans le composé de formule générale L. Les composés de formule générale L peuvent être transformés en pyridones de formule générale M, par exemple, par un traitement à l'acide chlorhydrique à chaud, ou par un traitement à l'iodure de triméthylsilyle.

Les 2-halo-3-quinoléine-méthanols de formule générale N peuvent être obtenus à partir d'acétanilides de formule générale P 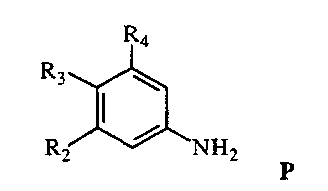 dans laquelle R₂, R₃ et R₄ ont la signification indiquée dans les formules générales des composés I et II. Dans les procédés ci-dessous, les groupes R₂, R₃ et R₄ peuvent être protégés si nécessaire selon les méthodes classiques de protection (Greene, T., Protective Groups in Organic Synthesis 10-86 (John Wiley & Sons 1981)).

Les composés de formule N peuvent donc être obtenus selon le procédé suivant : les dites anilines de formule P sont *N*-acétylées par traitement avec un agent acétylant tel que par exemple l'anhydride acétique. Les acétanilides ainsi obtenus sont traités à une température comprise entre 50° C et 100° C, de préférence 75° C, par un réactif connu de l'homme de l'art sous de nom de réactif de Vilsmeyer (obtenu par l'action de l'oxychlorure de phosphoryle sur la *N,N*-diméthylformamide à une température comprise entre 0° C et 10° C) pour donner le 2-chloro-3-quinoléinecarbaldéhyde correspondant (se référer, par exemple à Meth-Cohn, et coll. *J. Chem. Soc., Perkin Trans. I* p.1520 (1981); Meth-Cohn, et coll. *J. Chem. Soc., Perkin Trans. I* p.2509 (1981); et Nakasimhan et coll. *J. Am. Chem. Soc., 112*, p.4431 (1990)). Le chlore en position 2 des 2-chloro-3-quinoléinecarbaldéhydes peut être substitué par de l'iode ou du brome en chauffant le produit dans un solvant inerte tel que l'acétonitrile en présence d'un sel d'iode ou de brome (par exemple l'iodure de sodium ou le bromure de tétrabutylammonium). Une trace d'acide tel que l'acide chlorhydrique concentré peut être nécessaire pour catalyser cette transformation. Les 2-halo-3-quinoléinecarbaldéhydes sont facilement réduits en 2-halo-3-quinoléineméthanols correspondants de formule générale N, dans des conditions classiques connues de l'homme de l'art telles que le traitement dans un solvant alcoolique (par exemple le méthanol) par du borohydrure de sodium à une température comprise entre 0° C et 40° C.

Les composés de formule N peuvent également être obtenus selon le procédé suivant : les anilines de formule générale P telles que définies ci-dessus sont acylées par réaction avec un nitrile (tel que le chloroacétonitrile ou le propionitrile) en présence de trichlorure de bore et d'un autre acide de Lewis tel que le trichlorure d'aluminium, le tétrachlorure de titane ou le chlorure de diéthylaluminium dans un solvant aprotique ou un mélange de solvant aprotique, suivie d'une hydrolyse (*cf.* Sugasawa, T, et coll. *J. Am. Chem. Soc. 100*, p. 4842 (1978)). L'intermédiaire ainsi obtenu est ensuite traité par le chlorure d'éthylmalonyle dans un solvant aprotique tel que l'acétonitrile en présence d'une base telle que la triéthylamine, puis traité par un alcoolate alcalin, par exemple le méthylate de sodium dans le méthanol, pour donner un 2-hydroxy-3-quinoléinecarboxylate d'éthyle substitué en position 4. Ce dernier est transformé en 2-chloro-3-quinoléinecarboxylate d'éthyle par un traitement avec de l'oxychlorure de phosphoryle. Lorsque la position 4 de la quinoléine porte un groupe chlorométhyle, on peut effectuer une substitution nucléophile par traitement avec une amine secondaire telle que par exemple la diméthylamine, la N-méthylpipérazine, la morpholine ou la pipéridine. Le 2-chloro-3-quinoléinecarboxylate d'éthyle est ensuite réduit par l'hydrure de diisobutylaluminium dans un solvant aprotique que le dichlorométhane pour donner le 2-chloro-3-quinoléineméthanol de formule générale N. Des analogues des composés intermédiaires (N) ont été décrits dans la littérature et en particulier dans la demande PCT 95/05427.

Certains composés de l'invention peuvent être préparés sous la forme de sels pharmaceutiquement acceptables selon les méthodes usuelles. Des sels acceptables comprennent, à titre d'exemple et de façon non limitative, des sels d'addition d'acides inorganiques tels que chlorhydrate, sulfate, phosphate, diphosphate, bromhydrate et nitrate ou d'acides organiques tels que acétate, maléate, fumarate, tartrate, succinate, citrate, lactate, méthane sulfonate, p-toluènesulfonate, pamoate, salicylate, oxalate et stéarate. Entrent également dans le champ d'application de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Pharmaceutical Salts", J. Pharm. Sci. 66:1 (1977).

Les composés de la présente invention possèdent d'intéressantes propriétés pharmacologiques. C'est ainsi que les composés de la présente invention ont une activité inhibitrice de la topoisomérase I et une activité anti-tumorale. L'état de la technique suggère que les composés de l'invention présentent une activité anti-parasitique et/ou anti-virale. Les composés de la présente invention peuvent ainsi être utilisés dans différentes applications thérapeutiques.

On trouvera ci-après, dans la partie expérimentale, une illustration des propriétés pharmacologiques des composés de l'invention.

Les composés peuvent inhiber la topoisomérase, par exemple de type I, chez un patient, par exemple un mammifère tel que l'homme, par administration à ce patient d'une quantité thérapeutiquement efficace d'un composé de formule (I).

Les composés de l'invention possèdent également une activité anti-tumorale. Ils peuvent être utilisés pour le traitement des tumeurs, par exemple des tumeurs exprimant une topoisomérase, chez un patient par administration à ce dernier d'une quantité thérapeutiquement efficace d'un composé de formule (I). Des exemples de tumeurs ou de cancers comprennent les cancers de l'oesophage, de l'estomac, des intestins, du rectum, de la cavité orale, du pharynx, du larynx, du poumon, du colon, du sein, du cervix uteri, du corpus endometrium, des ovaires, de la prostate, des testicules, de la vessie, des reins, du foie, du pancréas, des os, des tissus conjonctifs, de la peau, des yeux, du cerveau et du système nerveux central, ainsi que le cancer de la thyroïde, la leucémie, la maladie de Hodgkin, les lymphomes autres que ceux de Hodgkin, les myélomes multiples et autres.

Ils peuvent également être utilisés pour le traitement des infections parasitiques par l'inhibition des hémoflagellates (par exemple dans la trypanosomie ou les infections leiskmania) ou par inhibition de la plasmodie (comme par exemple dans la malaria), mais aussi le traitement des infections ou maladies virales.

Ces propriétés rendent les produits de formule (I) aptes à une utilisation pharmaceutique. La présente demande a également pour objet, à titre de médicaments, les produits de formule (I) telles que définies ci-dessus, ainsi que les sels d'addition avec les acides minéraux ou organiques pharmaceutiquement acceptables desdits produits de formule (I), ainsi que les compositions pharmaceutiques contenant, à titre de principe actif, l'un au moins des médicaments tels que définis ci-dessus.

L'invention concerne ainsi des compositions pharmaceutiques contenant un composé de l'invention ou un sel additif d'acide pharmaceutiquement acceptable de celui-ci, en association avec un support pharmaceutiquement acceptable suivant le mode d'administration choisie (par exemple orale, intraveineuse, intrapéritonéales, intramusculaires, trans-dermique ou sous-cutanée). La composition pharmaceutique (par exemple thérapeutique) peut être sous forme solide, liquide, de liposomes ou de micelles lipidiques.

La composition pharmaceutique peut être sous forme solide comme, par exemple, les poudres, pilules, granules, comprimés, liposomes, gélules ou suppositoires. La pilule, le comprimé ou la gélule peuvent être revêtus d'une substance capable de protéger la composition de l'action de l'acide gastrique ou des enzymes dans l'estomac du sujet pendant une période de temps suffisante pour permettre à cette composition de passer non digérée dans l'intestin grêle de ce dernier. Le composé peut aussi être administré localement, par exemple à l'emplacement même de la tumeur. Le composé peut aussi être administré selon le processus de la libération prolongée (par exemple une composition à libération prolongée ou une pompe d'infusion). Les supports solides appropriés peuvent être, par exemple, le phosphate de calcium, le stéarate de magnésium, le carbonate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire. Les compositions pharmaceutiques contenant un composé de l'invention peuvent donc également se présenter sous forme liquide comme, par exemple, des solutions, des émulsions, des suspensions ou une formulation à libération prolongée. Les supports liquides appropriés peuvent être, par exemple, l'eau, les solvants organiques tels que le glycérol ou les glycols tel que le polyéthylène glycol, de même que leurs mélanges, dans des proportions variées, dans l'eau.

L'invention a également pour objet l'utilisation des produits de formule (I) telle que définie ci-dessus, pour la préparation de médicaments destinés à inhiber la topoisomèrase, de médicaments destinés à traiter les tumeurs, de médicaments destinés à traiter les infections parasitiques, ainsi que de médicaments destinés traiter des infections ou maladies virales.

La dose d'un composé selon la présente invention, à prévoir pour le traitement des maladies ou troubles mentionnés ci-dessus, varie suivant le mode d'administration, l'âge et le poids corporel du sujet à traiter ainsi que l'état de ce dernier, et il en sera décidé en définitive par le médecin ou le vétérinaire traitant. Une telle quantité déterminée par le médecin ou le vétérinaire traitant est appelée ici "quantité thérapeutiquement efficace".

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnés ici sont incorporés par référence.

Les exemples suivants sont présentés pour illustrer les procédures ci-dessus et ne doivent en aucun cas être considérés comme une limite à la portée de l'invention.

### PARTIE EXPÉRIMENTALE :

### Préparation 1 : 5-éthyl-4,5-dihydro-5-hydroxy-1H-oxépino [3',4':6,7]-indolizine [1,2-b] quinoléine-3,15 (4H,13H)-dione

### 1.a. 4-éthyl-3,4-dihydroxy-1H-pyrano [3',4':6,7] indolizino [1,2-b] quinoléine-14 (4H,12H)-one

On ajoute par portions du borohydrure de sodium (14 g, 370 mmol) à une suspension de (*S*)-(+)-camptothécine (14 g, 40 mmol, que l'on peut obtenir de différentes sources commerciales telles que Aldrich Chemical Co. (Milwaukee, WI)), dans le méthanol (750 ml) et on chauffe doucement le mélange résultant jusqu'à 55° C afin d'obtenir une solution limpide que l'on agite ensuite pendant 16 heures à la température ambiante. Le solvant est ensuite évaporé sous pression réduite, le résidu est repris dans l'eau (250 ml), neutralisé par addition d'acide acétique (21 ml) et laissé au repos 2 heures à 4° C. La suspension résultante est filtrée et lavée successivement avec de l'eau froide, de l'acétone et du diéthyl éther, ce qui permet d'obtenir après séchage sous pression réduite le composé recherché sous forme d'un solide blanc, p.f. 280° C.

### 1.b. 8-formyloxyméthyl-7-propionylindolizino [1,2-b] quinoléine-9 (11H)-one

On ajoute goutte à goutte une solution de métaperiodate de sodium (14 g, 65 mmol) dans l'eau (140 ml) à une suspension de 4-éthyl-3,4-dihydroxy-1H-pyrano [3', 4':6,7] indolizino [1,2-*b*] quinoléine-14 (4*H*,12*H*)-one (13,4 g, 38 mmol) dans de l'acide acétique glacial (720 ml) et on agite la solution résultante pendant une heure à température ambiante. Le mélange réactionnel est ensuite versé dans un mélange glace/eau (650 ml) et la suspension résultante est agitée pendant une demi-heure puis filtrée et lavée successivement avec de l'eau, de l'alcool isopropylique et du étherdiéthylique, ce qui permet d'obtenir, après séchage sous pression réduite, le composé recherché (11,5 g) sous forme d'un solide jaune pâle, p.f. > 200° C (d).

### 1.c. β-éthyl-β-hydroxy-β-(8-hydroxyméthyl-9-oxo (11H)-indolizino-[1,2-b] quinoléine-7-yl)-propionate de tert-butyle

Une suspension de zinc (6,5 g, 100 mmol) agitée avec un agitateur magnétique dans du diéthyléther anhydre (50 ml) sous argon, est activée, par addition goutte à goutte de chlorotriméthylsilane (0,75 ml, 5,7 mmol). On agite encore 15 minutes à température ambiante puis on chauffe à reflux. Le bain de chauffage est ensuite enlevé et du bromoacétate de tert-butyle (15 ml, 100 mmol) est ajouté goutte à goutte à une vitesse assurant le maintien au reflux. Le chauffage externe est remis et poursuivi pendant encore une heure. La solution éthérée résultante du réactif de Reformatsky est laissée à refroidir jusqu'à la température ambiante puis transférée au moyen d'une canule dans une suspension de 8-formyloxyméthyl-7-propionylindolizino [1,2-*b*] quinoléine-9 (11*H*)-one (1,6 g, 4,7 mmol) dans du tétrahydrofuranne anhydre (40 ml) sous argon. Le mélange réactionnel est agité au reflux pendant une heure, puis laissé à refroidir jusqu'à la température ambiante, et la réaction est arrêtée par ajout de chlorure d'ammonium saturé (100 ml) et on extrait avec du chloroforme (3 x 100 ml). Les extraits chloroformiques combinés sont séchés sur sulfate de sodium, évaporés, et le résidu est purifié par chromatographie sur colonne de gel de silice (1-2 % MeOH/CH₂Cl₂), ce qui permet d'obtenir 0,64 g du composé recherché (31 %) sous forme d'un solide jaune pâle, p.f. 146-149° C.
RMN-¹H (CDCl₃) : 0,93 (t, 3H) ; 1,37 (s, 9H) ; 1,99 (m, 2H) ; 2,97 (dd, 2H) ; 3,5 (se, 1H) ; 5,10 (s, 2H) ; 5,24 (s, 2H) ; 7,40 (s,1H) ; 7,59 (t, 1H) ; 7,83 (t, 1H) ; 7,90 (d, 1H) ; 8,20 (d, 1H) ; 8,34 (s, 1H).
RMN-¹³C (CDCl₃) : 8,18 ; 27,90 ; 34,59 ; 45,34 ; 49,91 ; 58,55 ; 77,39 ; 82,42 ; 100,52; 127,67 ; 127,97 ; 128,10 ; 128,64 ; 129,44; 129,79; 130,42 ; 130,99; 142,86 ; 148,69; 152,75 ; 155,16 ; 162,38 ; 172,24.
IR (KBr) : 764 ; 1016 ; 1157 ; 1580 ; 1651 ; 1726.

### 1.d. 5-éthyl-4,5-dihydro-5-hydroxy-1H-oxépino [3',4':6,7]-indolizine [1,2-b] quinoléine-3,15 (4H,13H)-dione

Du β-éthyl-β-hydroxy-β-(8-hydroxyméthyl-9-oxo (11*H*)-indolizino-[1,2-*b*] quinoléine-7-yl) propionate de *tert*-butyle (1,45 g, 3,32 mmol) est dissous dans du dichlorométhane anhydre (25 ml) et traité avec une solution saturée de chlorure d'hydrogène dans le dichlorométhane (100 ml). Le mélange résultant est maintenu à -20° C pendant 16 heures. Le précipité est filtré, lavé avec du méthanol et séché sous pression réduite, ce qui permet d'obtenir 662 mg (55 %) du composé recherché sous forme d'un solide jaune, p.f. > 300° C.
RMN-¹H (DMSO) : 0,90 (t, 3H) ; 1,20 (q, 2H) ; 3,27 (dd, 2H) ; 5,29 (s, 2H) ; 5,49 (dd, 2H) ; 7,42 (s, 1H) ; 7,73 (t, 1H) ; 7,90 (t, 1H) ; 8,16 (t, 2H) ; 8,71 (s, 1H).
RMN-¹³C (DMSO) : 8,45 ; 36,48 ; 42,54 ; 50,68 ; 61,44 ; 73,34 ; 99,78 ; 122,71 ; 127,83 ; 128,15 ; 128,75 ; 129,08 ; 130,07 ; 130,61 ; 131,81 ; 144,66 ; 148,04 ; 152,80 ; 155,91 ; 159,26 ; 172,08.
IR (KBr) : 761 ; 1127 ; 1204 ; 1285 ; 1580 ; 1653 ; 1757.

### Préparation 2 : résolution de la 5-éthyl-4,5-dihydro-5-hydroxy-1H-oxépino [3',4':6,7]-indolizino [1,2-b] quinoléine-3,15 (4H,13H)-dione

On porte à ébullition un mélange d'acide β-éthyl-β-hydroxy-(8-hydroxyméthylindolizino [1,2-*b*] quinoléine-9-(11*H*)-one-7-yl)-propionique (19,5 g, 51 mmol) et de L-(-)-α-méthylbenzylamine (12,12 g, 100 mmol) dans de l'éthanol absolu (1 l), on filtre à chaud et on laisse reposer 68 h. On filtre le précipité et on lave à l'éthanol et à l'éther pour donner 9,8 g d'un solide blanc. Une analyse par chromatographie liquide à haute pression sur phase stationnaire chirale ("HPLC chirale" sur colonne Chiral-AGP (Chromtech, Stockholm, Suède) 100 x 4 mm, éluant acétonitrile 2 % dans tampon phosphate 10 mM à pH 6,9, pics éluant à 4,5 et 7,5 min) révèle deux pics intégrant respectivement pour 24 % et 76 % de la surface totale des deux pics. On reprend le solide dans de l'éthanol à 93 % (350 ml) au reflux, puis on laisse reposer 48 h. On filtre le précipité puis on lave à l'éthanol et à l'éther pour obtenir 4,8 g d'un solide blanc donnant deux pics intégrant respectivement pour 9 % et 91 % de la surface totale des deux pics par HPLC chirale. On reprend le solide dans de l'éthanol à 50 % (48 ml) au reflux puis on laisse reposer 48 h. On filtre le précipité puis on lave à l'éthanol et à l'éther pour donner 2,7 g d'un solide blanc donnant deux pics intégrant respectivement pour 3 % et 97 % de la surface totale des deux pics par HPLC chirale. On reprend le solide dans de l'éthanol à 50 % (22 ml) au reflux puis on laisse reposer 48 h. On filtre le précipité puis on lave à l'éthanol et à l'éther pour obtenir 1,6 g d'un solide blanc donnant deux pics intégrant respectivement pour 1 % et 99 % de la surface totale des deux pics par HPLC chirale. On traite le sel résultant, diastéréoisomériquement enrichi, repris dans de l'eau distillée (20 ml), par de l'acide acétique (0,35 ml, 6,4 mmol) pendant 15 min. On filtre le précipité obtenu, on lave à l'eau, á l'acétone et à l'éther puis on sèche sous vide à 80° C pour obtenir 1,1 g d'un solide blanc. On reprend ce dernier dans de l'éthanol absolu (55 ml) additionné d'acide chlorhydrique concentré (11,5 N, 11 ml) pour obtenir une solution jaune qui est maintenue sous agitation à température ambiante 68 h. On filtre le précipité ainsi obtenu et on lave à l'eau, à l'éthanol et à l'éther, puis on sèche sous vide à 80° C pour obtenir 770 mg de 5-éthyl-4,5-dihydro-5-hydroxy-1*H*-oxépino [3',4':6,7]-indolizino [1,2-*b*] quinoléine-3,15 (4*H*,13*H*)-dione énantiomériquement enrichie. Une analyse par HPLC chirale (colonne Chiral-AGP, éluée par un gradient de 2 à 5 % d'acétonitrile dans tampon phosphate 10 mM à pH 6,9, pics éluant à 15 et 20 min) révèle un excès enantiomérique de 98 %. On reprend la procédure décrite ci-dessus en remplaçant la L-(-)-α-méthylbenzylamine par la D-(+)-α-méthylbenzylamine. On obtient ainsi l'autre énantiomère du 5-éthyl-4,5-dihydro-5-hydroxy-1*H*-oxépino [3',4':6,7]-indolizino [1,2-*b*] quinoléine-3,15 (4*H*,13*H*)-dione.

### Préparation 3 : 5,12-diéthyl-4,5-dihydro-5-hydroxy-1H-oxépino [3',4':6,7]-indolizino [1,2-b] quinoléine-3,15 (4H,13H)-dione

Ce composé est préparé d'une manière analogue à la préparation 1, excepté que dans l'étape 1.a., la 7-éthyl camptothécine (Sawada et ses collaborateurs, Chem. Pharm. Bull. 39:2574 (1991)) est utilisée à la place de la camptothécine. On obtient le composé recherché sous la forme d'un solide jaune vif, p.f. > 270° C.
RMN-¹H (DMSO) : 0,92 (t, 3H) ; 1,39 (t, 3H) ; 1,93 (q, 2H) ; 3,08 (d, 2H) ; 3,25 (q, 2H) ; 3,51 (d, 2H) ; 5,32 (s, 2H) ; 5,52 (dd, 2H) ; 7,42 (s, 1H) ; 7,76 (t, 1H) ; 7,89 (t, 1H) ; 8,18 (d, 1H) ; 8,32 (d, 1H).
RMN-¹³C (DMSO) : 8,46 ; 14,15 ; 22,42 ; 36,50 ; 42,54 ; 49,95 ; 61,45 ; 73,35 ; 99,68 ; 122,61 ; 124,27 ; 126,76 ; 127,70 ; 128,27 ; 129,92 ; 130,18 ; 145,17 ; 145,82 ; 148,57 ; 152,15 ; 155,89 ; 159,26 ; 172,08.

### Préparation 4 : 5-éthyl-9,10-difluoro-4,5-dihydro-5-hydroxy-1H-oxépino[3',4':6,7]indolizino[1,2-b]quinoléine-3,15(4H,13H)-dione

### 4.a. 2-éthyl-2-(2-méthoxy-4-pyridyl)-1,3-dioxolane

On distille de l'eau de manière azéotropique (une nuit entière) avec un appareil Dean Stark à partir d'un mélange de 2-chloro-4-propionylpyridine (10 g, 59 mmol) obtenue comme dans Lamattina, J.L. *J. Hétérocyclic Chem. 20*, p. 553 (1983), d'éthylène glycol (20 ml) et d'acide *p*-toluènesulfonique (250 mg) dans le toluène (150 ml). Le solvant est ensuite éliminé sous pression réduite, l'acide est neutralisé avec du bicarbonate de sodium aqueux saturé (100 ml) et le produit est extrait à l'éther. Les extraits éthérés combinés sont lavés avec de la saumure, séchés sur sulfate de sodium et évaporés, ce qui donne 13,3 g (96 %) de produit brut protégé par le groupe carbonyle qui est porté au reflux avec 3 équivalents de méthoxyde de sodium dans l'acétonitrile jusqu'à la fin de la réaction (contrôle par chromatographie sur couche mince : SiO₂, oxyde de *tert*-butyle méthyle / hexane (TBMO/HX) 50/50). La solution d'acétonitrile est ensuite filtrée et évaporée. Le résidu est repris dans l'éther, lavé avec de l'eau et de la saumure, séché sur sulfate de sodium et évaporé, ce qui donne une huile marron qui est distillée (70-75° C, 0,04 mbar) ; on récupère 10,7 g (rendement global 81 %) de produit (F) sous forme d'une huile limpide.

### 4.b. 2-éthyl-2-(3-hydroxyméthyl-2-méthoxy-4-pyridyl)-1,3-dioxolane

On ajoute goutte à goutte au moyen d'une canule du *tert*-butyllithium (1,7 *M* dans le pentane, 100 ml, 170 mmol) à une solution de bromomésitylène (13 ml, 85 mmol) dans du tétrahydrofurane anhydre (300 ml) à -78° C et sous argon. Le précipité blanc résultant est agité à -78° C pendant une heure puis du 2-éthyl-2-(2-méthoxy-4-pyridyl)-1,3-dioxolane (10 g, 44,8 mmol) est ajouté et le mélange réactionnel est agité 15 minutes à -78° C, une heure à 0° C et une heure à la température ambiante. Après un nouveau refroidissement à -78° C, du N,N-diméthylformamide anhydre (100 mmol) est ajoutée et le mélange réactionnel est laissé se réchauffer jusqu'à la température ambiante puis est agité pendant 16 heures, après quoi une analyse par chromatographie sur couche mince (SiO₂, TBMO/HX:50/50) fait apparaître la consommation complète du produit de départ. La réaction est arrêtée avec du chlorure d'ammonium saturé et le mélange réactionnel extrait avec du diéthyl éther (200 ml, 50 ml, 50 ml). Les extraits combinés sont séchés sur sulfate de sodium et évaporés, ce qui donne une huile jaune qui est purifiée par chromatographie sur colonne (SiO₂, TBMO/HX: 0/100 à 5/95 pour éluer les dérivés mésytylène puis 20/80 à 50/50 pour éluer le produit) pour obtenir l'aldéhyde intermédiaire (7 g). L'aldéhyde est dissous dans le méthanol (100 ml) et traité avec du borohydrure de sodium (5 g, 132 mmol) et le mélange résultant est agité jusqu'à consommation complète de l'aldéhyde intermédiaire (environ 1 heure) avec contrôle analytique par chromatographie sur couche mince. Le solvant est ensuite évaporé, le résidu est repris dans l'éther, lavé avec de l'eau et de la saumure, séché, et le solvant est évaporé. La chromatographie sur colonne (SiO₂, TBMO/HX: 10/90 à 50/50) du résidu donne 7 g (rendement global 62 %) de produit (G) sous forme d'une huile jaune.

### 4.c. 2-(3-benzyloxyméthyl-2-méthoxy-4-pyridyl)-2-éthyl-1,3-dioxolane

On ajoute goutte à goutte une solution de 2-éthyl-2-(3-hydroxyméthyl-2-méthoxy-4-pyridyl)-1,3-dioxolane (7 g, 30 mmol) et de chlorure de benzyle (5 ml, 45 mmol) dans du tétrahydrofurane anhydre (50 ml) à une suspension d'hydrure de sodium (80 % dans l'huile minérale, 1,85 g, 61 mmol) dans du tétrahydrofurane anhydre (100 ml) et on maintient au reflux le mélange réactionnel pendant 16 heures. Le mélange réactionnel est ensuite laissé à refroidir jusqu'à la température ambiante, la réaction est arrêtée avec de l'eau (50 ml) et le mélange réactionnel concentré sous pression réduite. Le résidu est dissous dans du diéthyl éther (150 ml) et lavé avec de l'eau et de la saumure, séché et évaporé. Une purification par chromatographie sur colonne (SiO₂, TBMO/HX: 5/95 à 20/80) donne le produit protégé par le benzyle (H), 9 g, (87 %) sous forme d'une huile limpide.

### 4.d. 1-(3-benzyloxyméthyl-2-méthoxy-4-pyridyl)-propane-1-one

On traite du 2-(3-benzyloxyméthyl-2-méthoxy-4-pyridyl)-2-éthyl-1,3-dioxolane (9 g, 27 mmol) avec de l'acide trifluoracétique (10 ml) et de l'eau (5 ml) à une température de bain de 120° C pendant 3 heures. Le mélange réactionnel est concentré sous pression réduite et les traces résiduelles d'acides sont neutralisées par l'addition de bicarbonate de sodium aqueux saturé. L'extraction avec de l'éther suivie d'une chromatographie sur colonne (SiO₂, TBMO/HX: 10/90) donne 5,5 g (70 %) de produit (I).

### 4.e. β-éthyl-β-hydroxy-β-(3-benzyloxyméthyl-2-méthoxy-4-pyridyl) propionate de tert-butyle

On ajoute goutte à goutte du bromoacétate de *tert*-butyle (13 ml, 80 mmol) à une suspension de zinc (5,3 g, 80 mmol activée par traitement avec HCl 6 *N* en 10 secondes, puis lavée successivement avec de l'eau jusqu'à pH neutre, de l'acétone et du diéthyl éther) dans le tétrahydrofurane anhydre (60 ml) au reflux. Le milieu réactionnel est maintenu au reflux pendant encore 10 minutes après que l'addition soit terminée. Ensuite, on ajoute une solution de 1-(3-benzyloxyméthyl-2-méthoxy-4-pyridyl)-propane-1-one (5,8 g, 20 mmol) dans le tétrahydrofurane anhydre (20 ml) et on agite le mélange réactionnel sous reflux pendant une heure supplémentaire. La réaction est arrêtée à 0° C avec du chlorure d'ammonium aqueux saturé (100 ml) et le mélange réactionnel extrait avec du diéthyl éther. Les extraits combinés sont séchés sur sulfate de sodium et évaporés, ce qui donne une huile jaune qui est purifiée par chromatographie sur colonne (SiO₂, TBMO/HX: 5/95 à 10/90) pour obtenir l'ester de *tert*-butyle (J) (7 g, 95 %) sous forme d'une huile limpide.

### 4.f. β-éthyl-β-hydroxy-β-(3-hydroxyméthyl-2-méthoxy-4-pyridyl) propionate de tert-butyle

On hydrogénolyse du β-éthyl-β-hydroxy-β-(3-benzyloxyméthyl-2-méthoxy-4-pyridyl) propionate de *tert*-butyle (1 g, 2,5 mmol) à la pression atmosphérique et à la température ambiante en utilisant 5 % de palladium sur charbon comme catalyseur (50 mg) et de l'éthanol absolu comme solvant (10 ml). Une fois la réaction terminée (6 heures), le catalyseur est séparé par filtration et le solvant est évaporé, ce qui laisse 0,7 g (90 %) de produit (K) d'une pureté suffisante pour une utilisation synthétique ultérieure.

### 4.g. 5-éthyl-1,5-dihydro-5-hydroxy-9-méthoxy-oxépino[3,4-c] pyridine-3(4H)-one

Du β-éthyl-β-hydroxy-β-(3-hydroxyméthyl-2-méthoxy-4-pyridyl) propionate de *tert*-butyle (8,8 g, 28 mmol) est traité avec de l'acide trifluoroacétique (30 ml) pendant 3 heures à la température ambiante. Les composants volatils sont évaporés et le résidu est purifié par chromatographie sur colonne (SiO₂, CH₂Cl₂/MeOH: 100/0 à 98/2), ce qui donne une huile limpide qui, après traitement avec du toluène, donne 5,9 g de produit (L) (89 %) sous forme de cristaux blancs, p.f. 97-98° C.

### 4.h. 5-éthyl-1,5-dihydro-5-hydroxy-oxépino[3,4-c]pyridine 3,9(4H,8H)-dione

On chauffe au reflux pendant 9 heures dans de l'acide chlorhydrique 1 N (20 ml) de la 5-éthyl-1,5-dihydro-5-hydroxy-9-méthoxy-oxépino [3,4-c] pyridine-3 (4*H*)-one (0,5 g, 2,1 mmol). Le mélange réactionnel est concentré sous pression réduite et le résidu est encore séché par addition et évaporation du toluène, par deux fois, puis laissé une nuit entière sous pression réduite en présence de pentoxyde de phosphore. L'huile résultante est dissoute dans l'acétonitrile anhydre (5 ml) et agitée sous argon pendant 24 heures. Le précipité est filtré et séché, ce qui donne 0,23 g (49 %) d'un solide blanc (M), p.f. 118-119° C.

### 4.i. 2-chloro-6,7-difluoro-3-quinoléine-méthanol

La procédure décrite par Meth-Cohn et ses collaborateurs, J. Chem. Soc. Perkin Trans. I, p. 1520 (1981) ; Meth-Cohn, J. Chem. Soc. Perkin Trans I, p. 2509 (1981) est employée. Du 3,4-difluoroacétanilide (38 g, 22 mmol) est ajouté au réactif de Vilsmeyer obtenu par addition goutte à goutte d'oxychlorure de phosphoryle (103 ml, 1,1 mol) à du diméthylformamide anhydre (34 ml, 44 mmol), refroidi avec un bain eau / glace et agitée pendant 0,5 heure sous atmosphère d'argon. Le mélange résultant est chauffé à 70° C pendant 16 heures. Après refroidissement à la température ambiante, le mélange réactionnel est ajouté à un mélange de glace et d'eau (400 ml) qui est maintenu sous agitation pendant 2 heures, puis filtré et lavé successivement à l'eau, à l'éthanol et à l'éther pour donner 9 g de 2-chloro-6,7-difluoroquinoléine-3-carbaldéhyde sous forme d'un solide jaune, p.f. 222-224° C. Cet intermédiaire est traité avec du borohydrure de sodium (2 g, 52 mmol) dans le méthanol (400 ml) à température ambiante pendant 0,5 heure puis l'exces de réatif est détruit par addition d'acide acétique (2 ml). Le solvant est éliminé sous pression réduite, le résidu est mis en solution dans de l'acétate d'éthyle et lavé successivement au bicarbonate de soudium dilué, à l'eau et avec une solution aqueuse saturée en chlorure de sodium. La phase organique est séchée sur sulfate de sodium, filtrée et concentrée. Le solide résultant est recristallisé dans le 1,2-dichloroéthane pour donner 8 g de 2-chloro-6,7-difluoro-3-quinoléine-méthanol sous forme d'un solide beige.

### 4.j. 5-éthyl-8-(2-chloro-6,7-difluoro-3-quinoléineméthyl)-1,5-dihydro-5-hydroxy-oxépino [3,4-c] pyridine-3,9(4H,8H)-dione

On ajoute goutte à goutte pendant 5 minutes de l'azodicarboxylate de diéthyle (570 µl, 3,6 mmol) à une solution de 5-éthyl-1,5-dihydro-5-hydroxy-oxépino[3,4-c]pyridine 3,9(4*H*,8*H*)-dione (400 mg, 1,79 mmol), du composé obtenu à l'étape précédente 4.i. (770 mg, 2,23 mmol) et de triphénylphosphine (934 mg, 3,58 mmol) dans du N,N-diméthylformamide anhydre (45 ml) et on agite le mélange résultant sous argon à la température ambiante pendant 16 heures. Le mélange réactionnel est ensuite concentré sous pression réduite et le résidu est dissous dans de l'éther (100 ml). La solution résultante est lavée avec de la saumure (4 x 50 ml), séchée sur sulfate de sodium et évaporée. Le résidu est purifié par chromatographie sur colonne (SiO₂, CH₂Cl₂/MeOH: 99/1 à 98/2), ce qui donne 650 mg (66 %) du produit (O) sous forme d'un solide blanc, p.f. 165-167° C.

### 4.k. 5-éthyl-9,10-difluoro-4,5-dihydro-5-hydroxy-1H-oxépino[3',4':6,7]indolizino[1,2-b]quinoléine-3,15(4H,13H)-dione

De la 5-éthyl-8-(2-chloro-6,7-difluoro-3-quinoléineméthyl)-1,5-dihydro-5-hydroxy-oxépino[3,4-c]pyridine-3,9(4*H*,8*H*)-dione (600 mg, 1,1 mmol), du bromure de tétrabutyl-ammonium (352 mg, 1,1 mmol), de l'acétate de sodium (359 mg, 4,4 mmol) et de l'acétate de palladium II (98 mg, 0,43 mmol) sont dissous dans de l'acétonitrile anhydre (40 ml) et chauffés à 90° C sous argon pendant 16 heures. Après refroidissement à la température ambiante, un précipité blanc est séparé de la solution rougeâtre. Ce précipité est filtré et séché sous pression réduite. Le produit brut est mis en suspension dans l'eau, filtré et séché sous pression réduite sur du pentoxyde de phosphore, ce qui donne 250 mg du composé recherché sous forme d'un solide beige, p.f. > 250° C.
RMN-¹H (DMSO) : 0,91 (t, 3H) ; 1,87 (m, 2H) ; 3,08 (d, 1H) ; 3,51 (d, 1H) ; 4,45 (s, 4H) ; 5,19 (s, 2H) ; 5,47 (dd, 2H) ; 6,02 (se, 1H) ; 7,33 (s, 1H) ; 7,54 (s,1H) ; 7,55 (s, 1H) ; 8,43 (s, 1H).
RMN-¹³C (DMSO) : 8,43 ; 36,47 ; 42,54 ; 50,52 ; 61,43 ; 64,43 (2C) ; 73,31 ; 99,07 ; 112,27 ; 113,14 ; 122,00 ; 124,24 ; 128,18 ; 129,74 ; 144,59 ; 145,01 ; 145,33 ; 147,63 ; 150,88 ; 155,88 ; 159,23 ; 172,07.

### Préparation 5 : 5-éthyl-4,5-dihydro-5,10-dihydroxy-1H-oxépino [3',4':6,7]-indolizino [1,2-b]quinoléine-3,15 (4H,13H)-dione

La 10-benzyloxy-5-éthyl-4,5-dihydro-5-hydroxy-1*H*-oxépino[3',4':6,7]-indolizino[1,2-b] quinoléine-3,15 (4*H*,13*H*)-dione (370 mg, 0,79 mmol) est traitée par hydrogène à la pression atmosphérique et à température ambiante en utilisant du palladium à 10 % sur du charbon comme catalyseur (60 mg) et de l'acide trifluoroacétique comme solvant (15 ml). Une fois la réaction terminée (16 heures), du dichlorométhane (50 ml) et du méthanol (50 ml) sont ajoutés au mélange réactionnel, le catalyseur est filtré et les composants volatils sont évaporés sous pression réduite, ce qui permet d'obtenir le composé recherché brut contenant des traces d'acide trifluoroacétique. Ces traces sont éliminées par co-distillation avec du 1,4-dioxane. On obtient le produit sous forme d'un solide orange, p.f. 150° C (d), d'une pureté suffisante pour une utilisation synthétique ultérieure.
RMN-¹H (DMSO) : 0,89 (t, 3H) ; 1,85 (q, 2H) ; 3,02 (d, 1H) ; 3,45 (d, 1H) ; 5,19 (s, 2H) ; 5,37 (d, 1H) ; 5,50 (d, 1H) ; 5,98 (se, 1H) ; 7,26 (s 1H) ; 7,31 (s, 1H) ; 7,40 (d, 1H) ; 8,00 (d, 1H) ; 8,42 (s, 1H) ; 10,32 (s, 1H).
RMN-¹³C (DMSO) : 8,47 ; 36,50 ; 42,61 ; 50,57 ; 61,46 ; 73,35 ; 98,84 ; 109,02 ; 121,83 ; 123,18 ; 129,50 ; 129,85 ; 130,12 ; 130,80 ; 143,39 ; 145,10 ; 149,69 ; 155,97 ; 156,82; 159,30 ; 172,11.

### Préparation 6 : 5-éthyl-9-fluoro-4,5-dihydro-5-hydroxy-10-méthoxy-1H-oxépino[3',4':6,7]indolizino[1,2-b]quinoléine-3,15(4H,13H)-dione

Ce composé est obtenu à partir de la 3-fluoro-4-méthoxyaniline selon la méthode illustrée par les étapes 4i, 4j et 4k de la préparation 4. Solide jaune, p.f. > 250° C.
RMN-¹H (DMSO) : 0,89 (t, 3H) ; 1,85 (q, 2H) ; 3,08 (d, 1H) ; 3,49 (d, 1H) ; 4,00 (s, 3H) ; 5,25 (s, 2H) ; 5,39 (d, 1H) ; 5,51 (d,1H) ; 6,00 (s, 1H) ; 7,32 (s, 1H) ; 7,72 (d, 1H) ; 7,91 (d, 1H) ; 8,58 (s, 1H).
RMN-¹³C (DMSO) : 8,43 ; 36,48 ; 42,51 ; 50,68 ; 56,60 ; 61,42 ; 73,29 ; 99,25 ; 108,68 ; 113,52 ; 122,23 ; 126,33 ; 129,99 ; 130,30 ; 143,79 ; 144,70 ; 148,42 ; 151,18 ; 153,19 ; 155,81 ; 159,20 ; 172,06. IR (KBr) : 1259 ; 1503 ; 1602 ; 1737.

### Préparation 7 : 9-chloro-5-éthyl-4,5-dihydro-5-hydroxy-10-méthyl-1H-oxépino[3',4':6,7]indolizino[1,2-b]quinoléine-3,15(4H,13H)-dione

Ce composé est obtenu à partir de la 3-chloro-4-méthoxyaniline selon la méthode illustrée par les étapes 4i, 4j et 4k de la préparation 4. Solide jaune ; p.f. > 250° C.
RMN-¹H (DMSO) : 0,85 (t, 3H) ; 1,85 (q, 2H) ; 2,55 (s, 3H) ; 3,07 (d, 1H) ; 3,45 (d, 1H) ; 5,25 (s, 2H) ; 5,39 (d, 1H) ; 5,51 (d, 1H) ; 6,05 (s, 1H) ; 7,39 (s, 1H) ; 8,10 (s, 1H) ; 8,20 (s, 1H) ; 8,60 (s, 1H).
RMN-¹³C (DMSO) : 8,43 ; 20,20 ; 36,47 ; 42,49 ; 50,67 ; 61,41 ; 73,28 ; 99,87 ; 122,82 ; 126,98 ; 127,99 ; 129,60 ; 130,53 ; 131,08 ; 135,64 ; 136,56 ; 144,39 ; 147,11 ; 153,10; 155,85 ; 159,18 ; 172,03.
IR (KBr) : 1208 ; 1479 ; 1606 ; 1656 ; 1724.

### Préparation 8 : 8-éthyl-2,3,8,9-tétrahydro-8-hydroxy-10H,12H-[1,4] dioxino [2,3-g] oxépino [3',4':6,7] indolizino [1,2-b] quinoléine-10,13 (15H)-dione

Ce composé est obtenu à partir de la 3,4-éthylènedioxyaniline selon la méthode illustrée par les étapes 4i, 4j et 4k de la préparation 4. Solide jaune ; p.f. > 250° C.
RMN-¹H (DMSO) : 0,85 (t, 3H) ; 1,85 (q, 2H) ; 3,07 (d, 1H) ; 3,47 (d, 1H) ; 5,25 (s, 2H) ; 5,39 (d, 1H) ; 5,51 (d, 1H) ; 6,05 (s, 1H) ; 7,39 (s, 1H) ; 8,15 (q, 1H) ; 8,25 (q, 1H) ; 8,68 (s, 1H).
RMN-¹³C (DMSO) : 8,41 ; 36,45 ; 42,48 ; 50,68 ; 61,40 ; 73,25 ; 99,92 ; 114,44 ; 115,42 ; 115,58 ; 122,96 ; 125,52 ; 130,56 ; 131,46 ; 144,21 ; 145,25 ; 142,36 ; 153,41 ; 155,85 ; 159,15 ; 172,00.
IR (KBr) : 1266 ; 1512 ; 1581 ; 1618 ; 1751.

### Préparation 9 : 7-éthyl-7,8-dihydro-7-hydroxy-9H,11H-[1,3] dioxolo [4,5-g] oxépino [3',4':6,7] indolizino [1,2-b] quinoléine-9,12[14H]-dione

Ce composé est obtenu à partir de la 3,4-méthylènedioxyaniline selon la méthode illustrée par les étapes 4i, 4j et 4k de la préparation 4. Solide crème ; p.f. >250° C.
RMN-¹H (DMSO) : 0,85 (t, 3H) ; 1,85 (q, 2H) ; 3,07 (d, 1H) ; 3,45 (d, 1H) ; 5,20 (s, 2H) ; 5,39 (d, 1H) ; 5,51 (d, 1H) ; 6,00 (s, 1H) ; 6,30 (s, 2H) ; 7,30 (s, 1H) ; 7,49 (d, 2H) ; 8,45 (s, 1H).
RMN-¹³C (DMSO) : 8,43 ; 36,49 ; 42,56 ; 50,58 ; 61,42 ; 73,31 ; 98,87 ; 102,75 ; 103,33 ; 104,92 ; 121,76 ; 125,74 ; 128,59 ; 130,33 ; 145,08 ; 146,69 ; 148,78 ; 150,19 ; 151,49 ; 155,90 ; 159,24 ; 172,08.
IR (KBr) : 1248 ; 1459 ; 1606 ;1731.

### Préparation 10 : 9-chloro-5-éthyl-4,5-dihydro-5-hydroxy-10-méthoxy-1H-oxépino [3',4':6,7] indolizino [1,2-b] quinoléine-3,15(4H,13H)-dione

Ce composé est obtenu à partir de la 3-chloro-4-méthoxyaniline selon la méthode illustrée par les étapes 4i, 4j et 4k de la préparation 4. Solide blanc ; p.f. > 250° C.
RMN-¹H (DMSO) : 0,85 (t, 3H) ; 1,85 (q, 2H) ; 3,07 (d, 1H) ; 3,45 (d, 1H) ; 4,01 (s, 3H) ; 5,22 (s, 2H) ; 5,39 (d, 1H) ; 5,51 (d, 1H) ; 6,02 (s, 1H) ; 7,31 (s, 1H) ; 7,68 (s, 1H) ; 8,20 (s, 1H) ; 8,55 (s, 1H).
RMN-¹³C (DMSO) : 8,22 ; 36,27 ; 42,30 ; 50,48 ; 56,69 ; 61,23 ; 73,08 ; 99,16 ; 107,44 ; 122,16 ; 127,12 ; 128,12 ; 129,25 ; 130,02 ; 130,53 ; 143,29 ; 144,37 ; 151,12 ; 153,29 ; 155,71 ; 158,98 ; 171,84.
IR (KBr) : 1056 ; 1256 ; 1483 ; 1592 ; 1657 ; 1747.

### Préparation 11 : 5-éthyl-4,5-dihydro-5-hydroxy-10-méthoxy-1H-oxépino [3',4':6,7] indolizino [1,2-b] quinoléine-3,15(4H,13H)-dione

Ce composé est obtenu à partir de la 4-méthoxyaniline selon la méthode illustrée par les étapes 4.i., 4.j. et 4.k. de la préparation 4. Solide jaune ; p.f. > 250° C.
RMN-¹H (DMSO) : 0,85 (t, 3H) ; 1,85 (q, 2H) ; 3,07 (d, 1H) ; 3,45 (d, 1H) ; 3,95 (s, 3H) ; 5,28 (s, 2H) ; 5,40 (d, 1H) ; 5,51 (d, 1H) ; 6,00 (s, 1H) ; 7,38 (s, 1H) ; 7,51 (d, 2H) ; 8,07 (d, 1H) ; 8,55 (s, 1H).
RMN-¹³C (DMSO) : 8,45 ; 36,48 ; 42,51 ; 50,64 ; 55,92 ; 61,42 ; 73,33 ; 99,01 ; 106,49; 122,02 ; 123,19 ; 129,59; 130,20 ; 130,43 ; 144,17; 144,94; 150,40; 155,92 ; 158,31 ; 159,26 ; 172,07.
IR (KBr) : 1251 ; 1604 ; 1655 ; 1735.

### Préparation 12 : 9,11 -dichloro-5-éthyl-4,5-dihydro-5-hydroxy-1H-oxépino[3',4':6,7]indolizino[1,2-b]quinoléine-3,15(4H,13H)-dione

Ce composé est obtenu à partir de la 3,5-dichloroaniline selon la méthode illustrée par les étapes 4.i., 4.j. et 4.k. de la préparation 4. Solide jaune ; p.f. > 250° C.
RMN-¹H (DMSO) : 0,85 (t, 3H) ; 1,85 (q, 2H) ; 3,07 (d, 1H) ; 3,45 (d, 1H) ; 5,30 (s, 2H) ; 5,41 (d, 1H) ; 5,55 (d, 1H) ; 6,08 (s, 1H) ; 7,41 (s, 1H) ; 8,05 (s, 1H) ; 8,21 (s, 1H) ; 8,91 (s, 1H).
RMN-¹³C (DMSO) : 8,39 ; 36,45 ; 42,51 ; 51,03 ; 61,39 ; 73,25 ; 100,62 ; 123,55 ; 124,63 ; 127,60 ; 128,08 ; 128,56 ; 132,06 ; 132,19 ; 134,53 ; 143,77 ; 148,80; 154,88 ; 155,82 ; 159,13 ; 171,98.
IR (KBr) : 1064 ; 1275 ; 1586 ; 1651 ; 1743.

### Préparation 13 : 5-éthyl-9-fluoro-4,5-dihydro-5-hydroxy-10-méthyl-1H-oxépino [3',4':6,7] indolizino [1,2-b]quinoléine-3,15(4H,13H)-dione

Ce composé est obtenu à partir de la 3-fluoro-4-méthylaniline selon la méthode illustrée par les étapes 4.i., 4.j. et 4.k. de la préparation 4. Solide jaune ; p.f. > 250° C.
RMN-¹H (DMSO) : 0,89 (t, 3H) ; 1,85 (q, 2H) ; 2,49 (s, 3H) ; 3,08 (d, 1H) ; 3,49 (d, 1H) ; 5,21 (s, 2H) ; 5,39 (d, 1H) ; 5,51 (d,1H) ; 6,05 (s, 1H) ; 7,39 (s, 1H) ; 7,87 (d, 1H) ; 8,05 (d, 1H) ; 8,61 (s, 1H).
RMN-¹³C (DMSO) : 8,40 ; 15,14 ; 36,45 ; 42,52 ; 50,60 ; 61,41 ; 73,28 ; 99,71 ; 112,00 ; 122,66 ; 125,38 ; 127,66 ; 129,59 ; 130,28 ; 144,49 ; 147,88 ; 152,88 ; 155,85 ; 159,18 ; 162,25 ; 172,02.
IR (KBr) : 1054 ; 1580 ; 1651 ; 1760.

### Préparation 14 : 5-éthyl-10-fluoro-4,5-dihydro-5-hydroxy-1H-oxépino [3',4':6,7] indolizino[1,2-b]quinoléine-3,15(4H,13H)-dione

Ce composé est obtenu à partir de la 4-fluoroaniline selon la méthode illustrée par les étapes 4.i., 4.j. et 4.k. de la préparation 4. Solide blanc ; p.f. > 250° C.
RMN-¹H (DMSO) : 0,85 (t, 3H) ; 1,85 (q, 2H) ; 3,07 (d, 1H) ; 3,45 (d, 1H) ; 5,29 (s, 2H) ; 5,39 (d, 1H) ; 5,55 (d, 1H) ; 6,30 (s, 1H) ; 7,39 (s, 1H) ; 7,80 (q, 1H) ; 7,99 (q, 1H) ; 8,23 (q, 1H) ;8,68 (s, 1H).
RMN-¹³C (DMSO) : 8,40 ; 36,46 ; 42,48 ; 50,66 ; 61,41 ; 73,31 ; 99,68 ; 111,83 ; 122,75 ; 128,93 ; 130,93 ; 131,22 ; 131,93 ; 144,46 ; 145,27 ; 152,60 ; 155,89 ; 159,21 ; 172,04.
IR (KBr) : 1209 ; 1589 ; 1659 ; 1739.

### Préparation 15 : 10-chloro-5-éthyl-4,5-dihydro-5-hydroxy-1H-oxépino [3',4':6,7]indolizino[1,2-b]quinoléine-3,15(4H,13H)-dione

Ce composé est obtenu à partir de la 4-chloroaniline selon la méthode illustrée par les étapes 4.i., 4.j. et 4.k. de la préparation 4. Solide jaune. p.f. > 250° C.
RMN-¹H (DMSO) : 0,85 (t, 3H) ; 1,85 (q, 2H) ; 3,07 (d, 1H) ; 3,47 (d, 1H) ; 5,25 (s, 2H) ; 5,39 (d, 1H) ; 5,51 (d, 1H) ; 6,05 (s, 1H) ; 7,39 (s, 1H) ; 7,89 (d, 1H) ; 8,19 (d, 1H) ; 8,29 (s, 1H) ; 8,67 (s, 1H).
RMN-¹³C (DMSO) : 8,40 ; 36,46 ; 42,47 ; 50,70 ; 61,42 ; 73,31 ; 100,00 ; 122,96 ; 127,31; 127,42 ; 128,87 ; 131,11 ; 132,12 ; 144,34 ; 146,53 ; 153,38 ; 155,88 ; 159,20 ; 172,04.
IR (KBr) : 1069 ;1483 ; 1606 ; 1741.

### Préparation 16 : 9-chloro-5-éthyl-10-fluoro-4,5-dihydro-5-hydroxy-1H-oxépino [3',4':6,7]indolizino[1,2-b]quinoléine-3,15(4H,13H)-dione

Ce composé est obtenu à partir de la 4-chloro-3-fluoroaniline selon la méthode illustrée par les étapes 4.i., 4.j. et 4.k. de la préparation 4. Solide jaune. p.f. > 250° C.
RMN-¹H (DMSO) : 0,85 (t, 3H) ; 1,85 (q, 2H) ; 3,07 (d, 1H) ; 3,45 (d, 1H) ; 5,25 (s, 2H) ; 5,39 (d, 1H) ; 5,51 (d, 1H) ; 6,05 (s, 1H) ; 7,40 (s, 1H) ; 8,20 (d, 1H) ; 8,40 (d, 1H) ; 8,68 (s, 1H).
RMN-¹³C (DMSO) : 8,38 ; 36,47 ; 42,58 ; 50,71 ; 61,40 ; 73,26 ; 99,99 ; 113,59 ; 123,09 ; 124,28 ; 127,74 ; 130,64 ; 131,31 ; 144,13 ; 145,08 ; 153,57 ; 154,13 ; 155,84 ; 156,61 ; 159,14 ; 172,00.
IR (KBr) : 1488 ; 1583 ; 1655 ; 1743.

### Préparation 17 : 5,12-diéthyl-9-fluoro-4,5-dihydro-5-hydroxy-10-méthoxy-1H-oxépino[3',4':6,7]indolizino[1,2-b] quinoléine-3,15(4H,13H)-dione

### 17.a. 5-fluoro-4-méthoxy-2-propionylaniline

(Ce produit est obtenu selon Sugasawa T; Toyoda T; Adachi M; Sasakura K, *J. Am. Chem. Soc*., 100 (1978), p4842-4852). A une solution de 3-fluoro-4-méthoxy-aniline (20 g, 142 mmol) dans du dichlorométhane anhydre (200 ml), sous atmosphère d'argon et à 0° C, est ajouté goutte à goutte du trichlorure de bore (1M dans l'heptane, 156 ml, 156 mmol). La suspension rose ainsi obtenue est maintenue sous agitation 5 min puis sont ajoutés goutte à goutte du propionitrile (33 ml, 420 mmol) suivi de trichlorure d'aluminium (20,8 g, 156 mmol) par petites portions. Le milieu réactionel est chauffé au reflux pendant 3 h, refroidi à 0° C, hydrolysé en ajoutant avec précaution de l'acide chlorhydrique 2N (100 ml), puis porté au reflux 45 min. Après refroidissement à 0° C, on obtient un précipité qui est filtré, lavé au dichloromethane, puis repris dans de l'eau (300 ml). La phase aqueuse est basifiée jusqu'à pH alcalin, extraite au dichlorométhane puis à l'acétate d'éthyle. La phase organique est séchée (MgSO₄) puis évaporée pour donner un produit brut qui est purifié par chromatographie sur colonne (SiO₂, AcOEt/Hpt: 1/99 à 20/80). On obtient 15,3 g d'un solide jaune.
RMN-¹H (CDCl₃) : 1,20 (t, 3H) ; 2,92 (q, 2H) ; 3,83 (s, 3H) ; 6,2 (s, 2H) ; 6,40 (d, 2H) ; 7,32 (d, 2H).
IR (KBr) : 857 ; 1148 ; 1240 ; 1561 ; 1583 ; 1662.

### 17.b. 4-éthyl-7-fluoro-2-hydroxy-6-méthoxy-3-quinoléinecarboxylate d'éthyle

A une solution de 5-fluoro-4-méthoxy-2-propionylaniline (15,3 g, 77,5 mmol) et de triéthylamine (13,9 ml, 100 mmol) dans de l'acétonitrile anhydre (110 ml), sous argon et à 0° C, est ajoutée goutte à goutte une solution de chlorure d'éthylmalonyle (12,9 ml, 100 mmol) dans de l'acétonitrile anhydre (30 ml). On laisse le milieu réactionel revenir à température ambiante, on canule goutte à goutte et sous argon une solution d'éthylate de sodium (obtenue par 1,8 g, 78 mmol, de sodium dans 80 ml d'éthanol), puis on laisse sous agitation 12 h à température ambiante. On verse le mélange réactionnel dans de l'eau glacée (100 ml) et on agite deux heures, puis on filtre le précipité qui est lavé à l'eau, à l'éthanol et à l'éther. On obtient 19,4 g d'un solide blanc.
RMN-¹H (DMSO) : 1,25 (m, 6H) ; 2,78 (q, 2H) ; 3,92 (s, 3H) ; 4,30 (q, 2H) ; 7,15 (d, 2H) ; 7,40 (d, 2H) ; 11,93 (s, 1H).
IR (KBr) : 786 ; 1083 ; 1410 ; 1521 ; 1644 ; 1725.

### 17.c. 2-chloro-4-éthyl-7-fluoro-6-méthoxy-3-quinoléinecarboxylate d'éthyle

On porte au reflux pendant 6 h une suspension de 4-éthyl-7-fluoro-2-hydroxy-6-méthoxy-3-quinoléinecarboxylate d'éthyle (19,4 g, 0,066 mol) dans du chlorure de phosphoryle (243 ml). On distille le chlorure de phosphoryle. On transvase le mélange réactionnel dans de l'eau glacée. On reprend au dichlorométhane pour solubiliser. On lave la phase organique à l'eau, puis avec une solution saturée de chlorure de sodium. On sèche la phase organique sur sulfate de magnésium et on évapore le solvant. On suspend le résidu dans de l'éther et filtre du produit de départ non converti (4 g). On évapore le filtrat et purifie le résidu par chromatographie sur colonne (SiO₂, AcOEt/Hpt: 5/95 à 20/80). On obtient 10,9 g d'un solide blanc.
RMN-¹H (DMSO) : 1,30 (t, 3H) ; 1,39 (t, 3H) ; 3,08 (q, 2H) ; 4,09 (s, 3H) ; 4,49 (q, 2H) ; 7,64 (d, 2H) ; 7,86 (d, 2H).
IR (KBr) : 865 ; 1016 ; 1082 ; 1190 ; 1224 ; 1253 ; 1272 ; 1508 ; 1571 ; 1732.

### 17.d. 2-chloro-4-éthyl-7-fluoro-6-méthoxy-3-quinoléineméthanol

Une solution de 2-chloro-4-éthyl-7-fluoro-6-méthoxy-3-quinoléinecarboxylate d'éthyle (10,8 g, 35 mmol) dans du dichlorométhane anhydre (200 ml) est traitée goutte à goutte à température ambiante sous atmosphère inerte par de l'hydrure de diisobutylaluminium (1M dans du dichlorométhane, 65 ml, 65 mmol), puis chauffée à 40° C pendant 4 h.

On refroidit à 0° C, on ajoute avec précaution une solution aqueuse de sel de Rochelle à 20 % (105 ml) et du dichlorométhane (200 ml) et on maintient sous agitation pendant 1 h. On décante, on lave trois fois à l'eau, on sèche la phase organique sur sulfate de magnésium et on évapore le solvant. Le résidu est purifié par chromatographie sur colonne (SiO₂, AcOEt/Hpt: 5/95 à 50/50). On obtient 6 g d'un solide blanc.
RMN-¹H (DMSO) : 1,28 (t, 3H) ; 3,25 (q, 2H) ; 4,04 (s, 3H) ; 4,77 (d, 2H) ; 5,27 (t, 1H) ; 7,55 (d, 2H) ; 7,73 (d, 2H).
IR (KBr) : 840 ; 864 ; 1023 ; 1232 ; 1267 ; 1317 ; 1444 ; 1511 ; 1569.

### 17.e. 5,12-diéthyl-9-fluoro-4,5-dihydro-5-hydroxy-10-méthoxy-1H-oxépino[3',4':6,7]indolizino[1,2-b]quinoléine-3,15(4H,13H)-dione

Le 2-chloro-4-éthyl-7-fluoro-6-méthoxy-3-quinoléineméthanol est couplé au composé (M) comme décrit dans l'étape 4.j. de la préparation 4. Le produit de couplage résultant est cyclisé selon la procédure de l'étape 4.k. On obtient un solide jaune, p.f. > 275° C.
RMN-¹H (CF₃COOD): 1,07 (m, 3H) ; 1,62 (m, 3H) ; 2,27 (m,2H) ; 3,44 (d, 1H) ; 3,54 (m, 2H) ; 3,91 (d, 1H) ; 4,25 (s, 3H) ; 5,60 (d, 1H) ; 5,74 (s, 2H) ; 5,98 (d, 1H) ; 7,85 (m, 1H) ; 8,16 (m, 1H) ; 8,31 (s, 1H).
RMN-¹³C (CF₃COOD): 9,03 ; 14,20 ; 26,68 ; 38,77 ; 43,98 ; 53,79 ; 58,27 ; 64,73 ; 77,93 ; 106,85 ; 109,24 ; 110,15 ; 128,99 ; 129,20 ; 131,61 ; 137,32 ; 141,23 ; 144,13 ; 154,79 ; 158,32 ; 160,25 ; 160,81 ; 179,30.
IR (KBr) : 1013 ; 1068 ; 1265 ; 1466 ; 1514 ; 1601 ; 1655 ; 1748.

### Préparation 18 : 5-éthyl-4,5-dihydro-5-hydroxy-12-méthyl-1H-oxépino [3',4':6,7]indolizino[1,2-b]quinoléine-3,15(4H,13H)-dione

On applique à la 2-acétylaniline la procédure décrite par les exemples 17.b., 17.c. et 17.d. pour donner du 2-chloro-4-méthyl-3-quinoléineméthanol. Ce dernier est couplé au composé (M) comme décrit dans l'étape 4.j. de la préparation 4. Le produit de couplage résultant est cyclisé selon la procédure de l'étape 4.k. On obtient un solide jaune, p.f. > 260° C.
RMN ¹H (DMSO) : 0,87 (t, 3H) ; 1,87 (q, 2H) ; 2,78 (s, 3H) ; 2,80 (d, 1H) ; 3,55 (d, 1H) ; 5,27 (s, 2H) ; 5,42 (d, 1H) ; 5,52 (d, 1H) ; 6,04 (s, 1H) ; 7,39 (s, 1H) ; 7,75 (t, 1H) ; 7,88 (t, 1H) ; 8,13 (d, 1H) ; 8,25 (d, 1H).
RMN-¹³C (DMSO) : 8,23 ; 36,26 ; 42,36 ; 62,00 ; 73,11 ; 78,65 ; 79,13 ; 79,25 ; 99,52 ; 122,36; 124,30; 127,67 ; 129,54; 129,55 ; 129,56; 140,11 ; 145,06 ; 148,07 ; 152,00 ; 155,79 ; 159,09 ; 171,89.
IR (KBr) : 1649 ; 1751 ; 3404.

### Préparation 19 : 10-benzyloxy-5-éthyl-9-fluoro-4,5-dihydro-5-hydroxy-1H-oxépino [3',4':6,7] indolizino [1,2-b] quinoléine-3,15(4H,13H)-dione

On applique au 3-fluoro-4-méthoxy-acétanilide la procédure exemplifiée dans l'étape 4.i. pour obtenir le 2-chloro-7-fluoro-6-méthoxy-quinoléine-3-carbaldéhyde que l'on traite par un excès de tribromure de bore dans le dichlorométhane à température ambiante pendant 24 heures. On obtient du 2-chloro-7-fluoro-6-hydroxy-quinoléine-3-carbaldéhyde, qui est O-benzylé dans le diméthylformamide en présence de bromure de benzyle et de carbonate de potassium pour donner du 6-benzyloxy-2-chloro-7-fluoroquinoléine-3-carbaldéhyde qui est réduit par du borohydrure de sodium dans le méthanol pour donner le quinoléineméthanol correspondant. Ce dernier est couplé au composé (M) comme décrit dans l'étape 4.j. de la préparation 4. Le produit de couplage résultant est cyclisé selon la procédure de l'étape 4.k. On obtient un solide jaune, p.f. > 275° C.
RMN-¹H (DMSO) : 0,86 (t, 3H) ; 1,85 (q, 2H) ; 3,05 (d, 1H) ; 5,25 (s, 2H) ; 5,37 (s, 2H) ; 5,45 (dd, 2H) ; 6,05 (s, 1H) ; 7,4-7,6 (m, 5H) ; 7.88 (d, 1H) ; 7,95 (d, 1H) ; 8,56 (s, 1H).

### Préparation 20 : 5-éthyl-9-fluoro-4,5-dihydro-5,10-dihydroxy-1H-oxépino [3',4':6,7] indolizino [1,2-b] quinoléine-3,15(4H,13H)-dione

On traite le composé de la préparation 19 (0,79 mmol) dissous dans de l'acide trifluoroacétique (15 ml) avec de l'hydrogène en utilisant du palladium à 10 % sur charbon (60 mg). On obtient un solide jaune, p.f. > 275° C.
RMN-¹H (DMSO) : 0,86 (t, 3H) ; 1,85 (q, 2H) ; 3,05 (d, 1H) ; 5,25 (s, 2H) ; 5,37 (s, 2H) ; 5,45 (dd, 2H) ; 6,05 (s, 1H) ; 7,8 (d, 1H) ; 7,90 (d, 1H) ; 8,56 (s, 1H).

Les préparations ci-dessus serviront de base pour l'illustration de l'invention par les exemples qui suivent.

### EXEMPLE 1 :

### 5-éthyl-9,10-difluoro-4,5-dihydro-5-(2-amino-1-oxoéthoxy)-1H-oxépino[3',4':6,7]indolizino[1,2-b]quinoléine-3,15(4H,13H)-dione

### a. 5-éthyl-9,10-difluoro-4,5-dihydro-5-(2-(t-butyloxycarbonylamino)-1-oxoéthoxy)-1H-oxépino[3',4':6,7]indolizino[1,2-b]quinoléine-3,15(4H,13H)-dione, chlorhydrate

Un mélange de 5-éthyl-9,10-difluoro-4,5-dihydro-5-hydroxy-1*H*-oxépino[3',4':6,7] indolizino[1,2-*b*]quinoléine-3,15(4*H*,13*H*)-dione (200 mg, 0,526 mmol, obtenu selon la préparation 4), de *N*-Boc-glycine (185 mg, 1,051 mmol) et d'une quantité catalytique de 4-diméthylaminopyridine (20 mg) dans de la pyridine anhydre (10 ml) est traité à 0° C et sous argon par du dicyclohexylcarbodiimide (239 mg, 1,16 mmol), puis agité à température ambiante pendant 48 heures. Les volatiles sont chassés sous vide et le résidue est chromatographié (SiO₂, 1 % méthanol dans le chloroforme) pour donner l'intermédiaire désiré (40 mg, 14 %), un solide jaune.
RMN-¹H (CDCl₃) : 1,20 (t, 3H) ; 1,38 (s, 9H) ; 1,40-1,70 (m, 2H) ; 3,10 (d, 1H) ; 4,00 (d, 2H) ; 4,30 (d, 1H) ; 5,00 (t, 1H) ; 5,20 (s, 2H) ; 5,30-5,90 (dd, 2H) ; 7,20 (s, 1H) ; 7,50-8,10 (m, 2H) ; 8,30 (s, 1H).

### b. 5-éthyl-9,10-difluoro-4,5-dihydro-5-(2-amino-1-oxoéthoxy)-1H-oxépino[3',4':6,7]indolizino[1,2-b]quinoléine-3,15(4H,13H)-dione, chlorhydrate

A l'intermédiaire obtenu ci-dessus (40 mg, 0,072 mmol) en solution dans du dichlorométhane (10 ml) et maintenu à 0° C est ajouté goutte à goutte du dioxane saturé en chlorure d'hydrogène (8 ml). La suspension jaune ainsi formée est maintenue sous agitation pendant 2 heures, puis les volatiles sont chassés sous vide. Le résidu, repris dans de l'eau (5 ml), est lavé au dichlorométane (3 x 30 ml). La phase aqueuse est congelée et lyophilisée pour donner le sel attendu, un solide jaune hygroscopique (20 mg, 50 %).
RMN-¹H (CDCl₃) : 1,00 (t, 3H) ; 2,15 (m, 1H) ; 2,30 (m, 1H) ; 3,60 (d, 1H) ; 3,90 (d, 1H) ; 4,15 (s, 2H) ; 5,10 (s, 2H) ; 5,40 (d, 1H) ; 5,70 (d, 2H) ; 7,40 (s, 1H) ; 7,80 (m, 2H) ; 8,50 (s, 1H).

### EXEMPLE 2 :

### 5-éthyl-9,10-difluoro-4,5-dihydro-5-(2-amino-1-oxopropoxy)-1H-oxépino[3',4':6,7]indolizino[1,2-b]quinoléine-3,15(4H,13H)-dione

On applique le procédé de l'exemple 1 à la 5-éthyl-9,10-difluoro-4,5-dihydro-5-hydroxy-1*H*-oxépino[3',4':6,7] indolizino[1,2-*b*]quinoléine-3,15(4*H*,13*H*)-dione en utilisant la *N*-Boc-*b*-alanine au lieu de la *N*-Boc-glycine, puis on clive le Boc protecteur de l'intermédiaire ainsi obtenu par un traitement par l'acide trifluoroacétique dans le dichlorométhane. On évapore les volatiles sous vide, on reprend le résidu dans le dichlorométhane. La solution résultante est lavée au bicarbonate dilué, séchée et évaporée. On obtient un solide jaune.

En appliquant la méthode des exemples 1 et 2 à d'autres composés, on obtient des résultats analogues. On a ainsi accès à toute une classe d'analogues de la camptothécine sous forme "prodrogue".

### EXEMPLE 3 :

### 1,8-diéthyl-8,9-dihydro-8-hydroxy-2H,10H,12H-[1,3]oxazino[5,6-f]oxépino[3',4':6,7]indolizino[1,2-b]quinoléine-10,13(15H)-dione :

Une suspension de 5-éthyl-4,5-dihydro-5,10-dihydroxy-1*H*-oxépino[3',4':6,7]indolizino[1,2-b] quinoléine-3,15 (4*H*,13*H*)-dione (84 mg obtenus selon la préparation 5) dans de l'acide acétique (2,5 ml) est traitée par de la 1,3,5-triéthylhexahydrotriazine (0,5 ml). Le mélange réactionnel est agité à 70° C pendant 30 min, puis évaporé sous vide. Le résidu est repris à l'éthanol, filtré et lavé à l'éther. On obtient un solide, p.f. > 275° C.
RMN-¹H (DMSO) : 0,87 (t, 3H) ; 1,50 (t, 3H) ; 1,85 (q, 2H) ; 2,77 (q, 2H) ; 3,05 (d, 1H) ; 3,47 (d, 1H) ; 4,37 (s, 2H) ; 5,00 (s, 2H) ; 5,22 (s, 2H) ; 5,45 (dd, 2H) ; 6,00 (s, 1H) ; 7,34 (s, 1H) ; 7,36 (d, 1H) ; 7,93 (d, 1H) ; 8,53 (s, 1H).
RMN-¹³C (DMSO) : 8,46 ; 13,48 ; 36,46 ; 42,49 ; 45,49 ; 46,44 ; 50,75 ; 61,43 ; 73,33 ; 82,06 ; 99,02 ; 112,90 ; 122,00 ; 122,98 ; 125,42 ; 127,04 ; 129,04 ; 130,20 ; 144,09 ; 144,97 ; 149,87 ; 152,92 ; 155,98 ; 172,07.
IR (KBr) : 1045 ; 1215 ; 1502 ; 1604 ; 1657 ; 1722.

### EXEMPLE 4 :

### 8-éthyl-8,9-dihydro-8-hydroxy-1-méthyl-2H,10H,12H-[1,3]oxazino[5,6-f]oxépino[3',4':6,7]indolizino[1,2-b]quinoléine-10,13(15H)-dione :

Une suspension de 5-éthyl-4,5-dihydro-5,10-dihydroxy-1*H*-oxépino [3',4':6,7]indolizino[1,2-*b*] quinoléine-3,15 (4*H*,13*H*)-dione (200 mg obtenus selon la préparation 5) dans de l'acide acétique (5 ml) est traitée par de la hexahydro-1,3,5-triméthyltriazine (110mg). Le mélange réactionnel est agité à 70° C pendant 30 min, puis évaporé sous vide. Le résidu est repris à l'éthanol, filtré et lavé à l'éther. On obtient un solide, p.f. > 275° C.
RMN-¹H (DMSO) : 0,87 (t, 3H) ; 1,85 (q, 2H) ; 3,04 (d, 1H) ; 3,48 (d, 1H) ; 4,33 (s, 2H) ; 4,93 (s, 2H) ; 5,28 (s, 2H) ; 5,45 (dd, 2H) ; 6,01 (s, 1H) ; 7,35 (s, 1H) ; 7,38 (d, 1H) ; 7,94 (d, 1H) ; 8,49 (s, 1H).
RMN-¹³C (DMSO) : 8,46 ; 36,43 ; 37,85 ; 42,55 ; 48,68 ; 50,79 ; 61,43 ; 73,35 ; 83,82 ; 99,04 ; 112,49 ; 122,04 ; 123,00 ; 125,46 ; 127,14 ; 129,07 ; 130,27 ; 144,99 ; 149,95 ; 152,46 ; 155,99 ; 172,09
IR (KBr) : 1047 ; 1058 ; 1219 ; 1246 ; 1295 ; 1439 ; 1504 ; 1604 ; 1655 ; 1735.

### EXEMPLE 5 :

### 8-éthyl-8,9-dihydro-8-hydroxy-1-benzyl-2H,10H,12H-[1,3]oxazino[5,6-f]oxépino[3',4':6,7]indolizino[1,2-b]quinoléine-10,13(15H)-dione :

Une suspension de 5-éthyl-4,5-dihydro-5,10-dihydroxy-1*H* -oxépino[3',4':6,7]indolizino[1,2-*b*] quinoléine-3,15 (4*H*,13*H*)-dione (200 mg obtenus selon la préparation 5) dans de l'acide acétique (5 ml) est traitée par de la 1,3,5-tribenzylhexahydrotriazine (285 mg). Le mélange réactionnel est agité à 70° C pendant 30 min, puis évaporé sous vide. Le résidu est repris à l'éthanol, filtré et lavé à l'éther. On obtient un solide, p.f. > 275° C.
RMN-¹H (DMSO) : 0,85 (t, 3H) ; 1,85 (q, 2H) ; 3,05 (d, 1H) ; 3,47 (d, 1H) ; 3,96 (s, 2H) ; 4,33 (s, 2H) ; 5,04 (s, 2H) ; 5,17 (s, 2H) ; 5,44 (dd, 2H) ; 6,01 (s, 1H) ; 7,38 (m, 6H) ; 7,42 (d, 1H) ; 7,97 (d, 1H) ; 8,42 (s, 1H).
RMN-¹³C (DMSO) : 8,42 ; 19,96 ; 36,45 ; 42,51 ; 46,36 ; 50,78 ; 55,38 ; 61,39 ; 73,31 ; 99,00 ; 112,55 ; 122,01 ; 123,08 ; 125,38 ; 127,09 ; 127,47 ; 128,70 ; 129,14 ; 130,35 ; 128,40 ; 139,19 ; 144,18 ; 149,99 ; 152,84 ; 155,92 ; 159,24 ; 172,05.
IR (KBr) : 1056 ; 1205 ; 1225 ; 1248 ; 1504 ; 1535 ; 1599 ; 1655 ; 1726.

### EXEMPLE 6 :

### 8-éthyl-8,9-dihydro-4-fluoro-8-hydroxy-1-benzyl-2H,10H,12H-[1,3]oxazino[5,6-f]oxépino[3',4':6,7]indolizino[1,2-b]quinoléine-10,13(15H)-dione :

Une suspension de 5-éthyl-9-fluoro-4,5-dihydro-5,10-dihydroxy-1*H*-oxépino[3',4':6,7]indolizino [1,2-b]quinoléine-3,15 (4*H*,13*H*)-dione (200 mg, obtenus selon la préparation 20) dans de l'acide acétique (5 ml) est traitée par de la 1,3,5-tribenzylhexahydrotriazine (285 mg). Le mélange réactionnel est agité à 70° C pendant 30 min, puis évaporé sous vide. Le résidu est repris à l'éthanol, filtré et lavé à l'éther. On obtient un solide, p.f. > 250° C.
RMN-¹H (DMSO) : 0,85 (t, 3H) ; 1,85 (q, 2H) ; 3,05 (d, 1H) ; 3,48 (d, 1H) ; 3,95 (s, 2H) ; 4,45 (s, 2H) ; 5,20 (s, 4H) ; 5,45 (dd, 2H) ; 6,05 (s, 1H) ; 7,40 (s, 7H) ; 7,90 (d, 1H) ; 8,45 (s, 1H).
IR (KBr) : 1248 ; 1451 ; 15001 ; 1598 ; 1657 ; 1727.

### Etude pharmacologique des produits de l'invention

### Test sur l'activité de relaxation de l'ADN induite par la topoisomérase 1.

Toutes les réactions sont effectuées dans un tampon de réaction de 20 µl constitué de 50 mM de Tris-HCl (pH 7,5), 50 mM de KCl, 0,5 mM de dithiothreitol, 10 mM de MgCl₂, 0,1 mM d'acide éthylènediamine tétraacétique (EDTA), 30 µg/ml d'albumine de sérum de bovin et 300 ng de pUC19 surenroulé (Pharmacia Biotech, Orsay, France) avec ou sans composés à tester à des concentrations définies. Tous les composés à tester sont dissous initialement dans le diméthylsulfoxyde (DMSO) ou dans l'eau pour les composés hydrosolubles, les autres dilutions ayant été faites dans l'eau distillée. La concentration en DMSO finale ne dépasse pas 1 % (v/v). La réaction est initiée par l'addition de topoisomérase 1 d'ADN de thymus de veau purifiée (Life technologies/Gibco-BRL, Paisley, Royaume-Uni) telle que la réaction soit complète en 15 minutes à 37° C. Les réactions sont arrêtées par addition de 3 µl d'un mélange contenant du sulfate de dodécyl sodium à 1 %, 20 mM d'EDTA et 500 µg/ml de protéinase K (Boehringer Mannheim, Meylan, France). Après une période d'incubation supplémentaire de 30 minutes à 37° C, 2 µl d'un tampon de chargement contenant 10 mM de Na₂HPO₄, du bleu de bromophénol à 0,3 % et du Ficoll à 16 % sont ajoutés aux échantillons qui sont soumis à une électrophorèse dans des gels d'agarose à 1,2 % à 1 V/cm pendant 20 heures dans un tampon contenant 36 mM de Tris-HCl pH 7,8, 30 mM de NaH₂PO₄, 1 mM d'EDTA et 2 µg/ml de chloroquine. Les gels sont colorés avec 2 µg/ml de bromure d'éthidium, photographiés sous lumière UV à 312 nm avec une caméra charge coupled device (ccd) et l'intensité fluorescente est mesurée à l'aide d'un analyseur d'image bioProfil (Vilber Lourmat, Lyon, France) en vue de déterminer le pourcentage d'ADN relaxé.

Dans chaque expérimentation, de l'ADN plasmidique surenroulé est incubé seul ou avec la topoisomérase 1. La réaction est complète en l'espace de 15 minutes. Pour chaque composé à tester ou témoin (on appelle témoin le véhicule seul), l'ADN plasmidique surenroulé est incubé en présence de la concentration maximale choisie pour l'expérience du composé à tester ou du témoin sans enzyme ou bien en présence du composé à tester, à des concentrations allant de 1 µM à 200 µM ou du témoin et en présence d'enzymes. Comme il est indiqué dans le Tableau I, les exemples 3 à 6 inhibent l'activité de relaxation favorisée par la topoisomérase 1 d'une manière dépendant de la concentration.

**TABLEAU I**

| | **Concentration micromolaire** | | | |
|---|---|---|---|---|
| | **10** | **50** | **100** | **200** |
| **Exemples** | | | | |
| **Camptothécine** | 88,7 | 62,4 | 52,9 | 46,9 |
| **3** | 79,7 | 46,9 | 33,5 | 23,2 |
| **4** | 86,2 | 32,7 | 35,1 | 32,1 |
| **5** | 56,2 | 30,4 | 28,0 | 24,2 |
| **6** | 55,6 | 39,9 | 38,9 | 30,0 |

## Revendications

1. Un composé **caractérisé en ce que** ledit composé est de formule (I) : 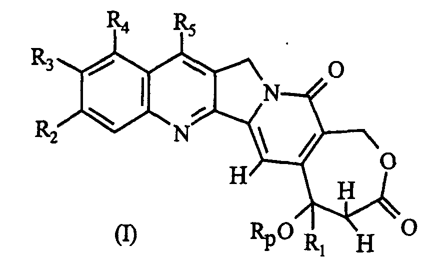 sous forme racémique, d'énantiomère ou toutes combinaisons de ces formes, dans laquelle
R₁ représente le groupe éthyle ;
R₂ représente H ou un halo ;
R₃ représente H, un alkyle inférieur, halo, ou OR₆ dans lequel R₆ représente H, un alkyle inférieur ou un aryle alkyle inférieur ;
R₄ représente H ou -(CH₂)ₘNR₆R₇ dans lequel R₆ et R₇ représentent, indépendamment, H ou un alkyle inférieur ;
R₅ représente H, un alkyle inférieur ou -(CH₂)ₙ[N≈X] substitué ou non substitué et [N≈X] représente le groupe pipéridyle, morpholinyle, pipérazinyle ou indolyle et ledit substituant est un alkyle inférieur ;
ou R₃ et R₄ forment un cycle oxazine éventuellement substitué sur l'atome d'azote par un radical R₉ qui représente un alkyle inférieur ;
Rₚ représente H ou un groupe facilement clivable de formule -C(O)-(A₁)-NH₂ dans lequel A₁ représente (CH₂)ₘ ou un radical alkylène inférieur ramifié ;
m est un nombre entier compris entre 0 et 6;
n est 1 ou 2; et
étant entendu que lorsque Rₚ est un atome d'hydrogène, R₃ et R₄ forment ensemble un cycle oxazine éventuellement substitué,
et lorsque le terme inférieur est associé à alkyle, il signifie au plus 6 atomes de carbone;
ou un sel pharmaceutiquement acceptable de ce dernier.

2. Un composé selon la revendication 1, **caractérisé en ce que** R₃ et R₄ forment un cycle oxazine éventuellement substitué ; ou un sel pharmaceutiquement acceptable de ce dernier.

3. Un composé selon la revendication 1, **caractérisé en ce que** Rₚ est un groupe facilement clivable ; ou un sel pharmaceutiquement acceptable de ce dernier.

4. Un composé selon la revendication 2, **caractérisé** en ce ledit composé est choisi parmi les composés répondant aux formules suivantes :
- 1,8-diéthyl-8,9-dihydro-8-hydroxy-2H,10H,12*H*-[1,3]oxazino[5,6-*f*]oxépino[3',4':6,7]indolizino[1,2-*b*]quinoléine-10,13(15*H*)-dione ;
- 8-éthyl-8,9-dihydro-8-hydroxy-1-méthyl-2*H*,10*H*,12*H*-[1,3]oxazino[5,6-*f*]oxépino[3',4':6,7]indolizino[1,2-*b*]quinoléine-10,13(15*H*)-dione ;
- 8-éthyl-8,9-dihydro-8-hydroxy-1-benzyl-2*H*,10*H*,12*H*-[1,3]oxazino[5,6-*f*]oxépino[3',4':6,7]indolizino[1,2-*b*]quinoléine-10,13(15*H*)-dione ;
- 8-éthyl-8,9-dihydro-4-fluoro-8-hydroxy-1-benzyl-2*H*,10*H*,12*H*-[1,3]oxazino[5,6-*f*]oxépino[3',4':6,7]indolizino[1,2-*b*]quinoléine-10,13(15*H*)-dione ;
ou un sel pharmaceutiquement acceptable de ce dernier.

5. Un composé selon la revendication 3, **caractérisé en ce que** ledit composé est choisi parmi les composés répondant aux formules suivantes :
- 5-éthyl-9,10-difluoro-4,5-dihydro-5-(2-amino-1-oxoéthoxy)- 1*H*-oxépino[3',4':6,7]indolizino[1,2-*b*]quinoléine-3,15(4*H*,13*H*)-dione ;
- 5-éthyl-9,10-difluoro-4,5-dihydro-5-(2-amine-1-oxopropoxy)-1*H*-oxépino[3',4':6,7]indolizino[1,2-*b*]quinoléine-3,15(4*H*,13*H*)-dione ;
ou un sel pharmaceutiquement acceptable de ce dernier.

6. A titre de médicament, un composé selon l'une quelconque des revendications précédentes ou un sel pharmaceutiquement acceptable de ce dernier.

7. Composition pharmaceutique contenant, à titre de principe actif, l'un au moins des composés selon l'une quelconque des revendications 1 à 5.

8. Utilisation d'un composé selon l'une quelconque des revendications 1 à 5 pour la préparation de médicaments antitumoraux.

9. Procédé de préparation de composés de formule Ia correspondant aux produits de formule I dans laquelle R₃ et R₄ forment un cycle oxazine selon l'une quelconque des revendications 1, 2 ou 4, **caractérisé en ce que**:
- l'on traite avec une amine primaire, dans les conditions de Mannich, un composé β-hydroxylactonique de formule générale D 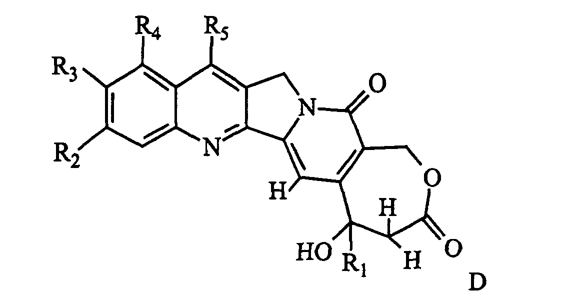
dans laquelle R₃ est un radical hydroxyle, R₄ est H, et R₁ et R₂ ont la signification indiquée ci-dessus pour obtenir un composé β-hydroxylactonique de formule générale Ia 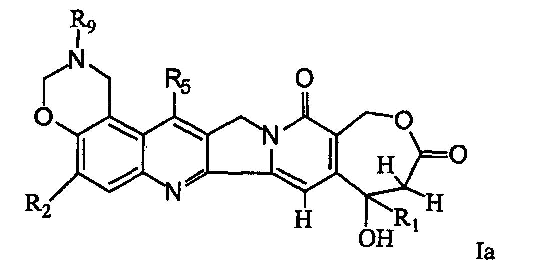 dans laquelle R₁, R₂, R₅ et R₉ ont la signification indiquée ci-dessus.

10. Procédé de préparation de composés de formule Ib correspondant aux produits de formule I dans laquelle Rₚ n'est pas un atome d'hydrogène, selon l'une quelconque des revendications 1, 2, 3 ou 5, **caractérisé en ce que** :
- l'on acyle le composé de formule générale D 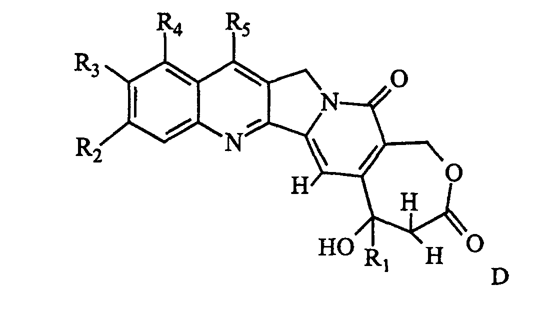
ou Ia 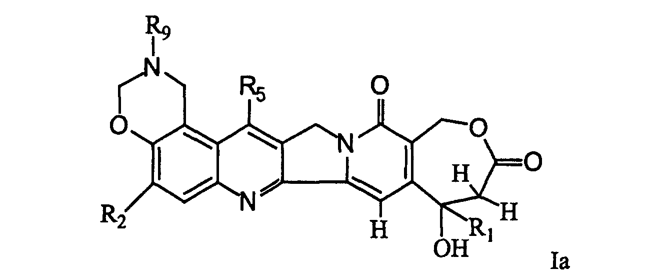 avec un dérivé du radical -C(O)-A₁-NH₂ comme défini dans la revendication 1, pour donner le composé β-hydroxylactonique de formule générale I avec Rₚ différent de H.

## Claims

1. A compound **characterized in that** said compound is of formula (I): 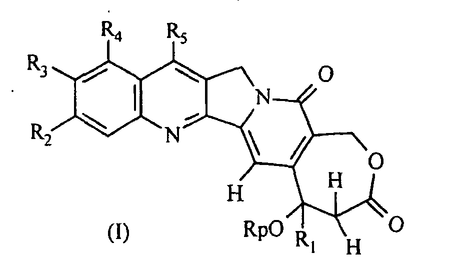 in racemic or enantiomeric form or any combinations of these forms, in which
R₁ represents the ethyl group ;
R₂ represents H or halo ;
R₃ represents H, lower alkyl, halo, or OR₆ in which R₆ represents H, lower alkyl or lower arylalkyl ;
R₄ represents H or -(CH₂)ₘNR₆R₇, in which R₆ and R₇ represent, independently, H or lower alkyl ;
R₅ represents H, lower alkyl or -(CH₂)ₙ[N≈X], substituted or unsubstituted and [N≈X] represents the piperidyl, morpholinyl, piperazinyl or indolyl group and said substituent is a lower alkyl ;
or R₃ and R₄ form an oxazine ring eventually substituted on the nitro atom by a R₉ radical which represents a lower alkyl ;
Rₚ represents H or an easily cleavable group of formula -C(O)-(A₁)-NH₂ in which A₁ represents (CH₂)ₘ or a branched lower alkylene radical;
m is an integer between 0 and 6 ;
n is 1 or 2 ; and
it being understood that when Rₚ is a hydrogen atom, R₃ and R₄ together form an eventually substituted oxazine ring,
and when the term lower is associated to alkyl or alkylene, it means no more than 6 carbon atoms;
or a pharmaceutically acceptable salt thereof.

2. A compound according to claim 1, **characterized in that** R₃ and R₄ form an optionally substituted oxazine ring ; or a pharmaceutically acceptable salt thereof.

3. A compound according to claim 1, **characterized in that** Rₚ is an easily cleavable group ; or a pharmaceutically acceptable salt thereof.

4. A compound according to claim 2, **characterized in that** said compound is chosen from the compounds corresponding to the following formulae :
- 1,8-diethyl-8,9-dihydro-8-hydroxy-2*H*,10*H*,12*H*-[1,3]oxazino[5,6-*f*]oxepino[3',4':6,7]indolizino[1,2-*b*]quinoline-10,13(15*H*)-dione ;
- 8-ethyl-8,9-dihydro-8-hydroxy-1-methyl-2*H*,10*H*,12*H*-[1,3]oxazino[5,6-*f*]oxepino[3',4':6,7]indolizino[1,2-*b*]quinoline-10,13(15*H*)-dione ;
- 8-ethyl-8,9-dihydro-8-hydroxy-1-benzyl-2*H*,10*H*,12*H*-[1,3]oxazino[5,6-*f*]oxepino[3',4':6,7]indolizino[1,2-*b*]quinoline-10,13(15*H*)-dione ;
- 8-ethyl-8,9-dihydro-4-fluoro-8-hydroxy-1-benzyl-2*H*,10*H*,12*H*-[1,3]oxazino[5,6-*f*]oxepino[3',4':6,7]indolizino[1,2-*b*]quinoline-10,13(15*H*)-dione ;
or a pharmaceutically acceptable salt thereof.

5. A compound according to claim 3, **characterized in that** said compound is chosen from the compounds corresponding to the following formulae :
- 5-ethyl-9,10-difluoro-4,5-dihydro-5-(2-amino-1-oxoethoxy)-1*H*-oxepino[3',4':6,7]indolizino[1,2-*b*]quinoline-3,15(4*H*,13*H*)-dione ;
- 5-ethyl-9,10-difluoro-4,5-dihydro-5-(2-amino- 1-oxopropoxy)-1*H*-oxepino[3',4':6,7]indolizino[1,2-*b*]quinoline-3,15(4*H*,13*H*)-dione ;
or a pharmaceutically acceptable salt thereof.

6. As a medicament, a compound according to any one of the previous claims or a pharmaceutically acceptable salt thereof.

7. Pharmaceutical composition containing, as active ingredient, at least one of the compounds according to any one of claims 1 to 5.

8. Use of a compound according to any one of claims 1 to 5 for the preparation of antitumoral medicaments.

9. Process for the preparation of compounds of formula Ia corresponding to the products of formula I in which R₃ and R₄ form an oxazine ring according to any one of claims 1, 2 or 4, **characterized in that**:
- a β-hydroxylactonic compound of general formula D 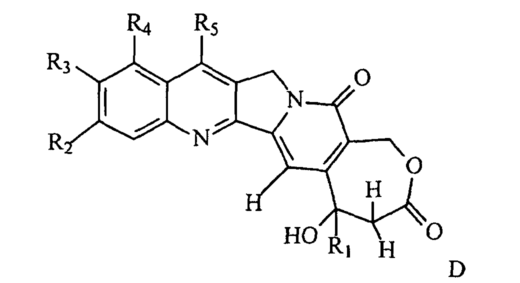
in which R₃ is a hydroxyl radical, R₄ is H, and R₁ and R₂ have the meaning indicated above is treated with primary amine, under Mannich's conditions, in order to obtain a β-hydroxylactonic compound of general formula Ia 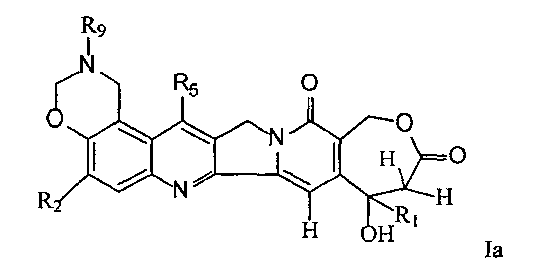 in which R₁, R₂, R₅ and R₉ have the meaning indicated above.

10. Process for the preparation of compounds of formula Ib corresponding to the products of formula I in which Rₚ is not a hydrogen atom, according to any one of claims 1, 2, 3 or 5, **characterized in that** :
- the compound of general formula D 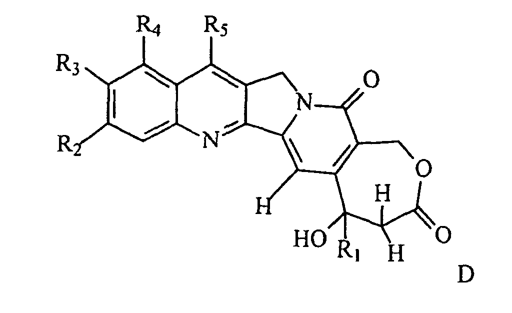 or Ia 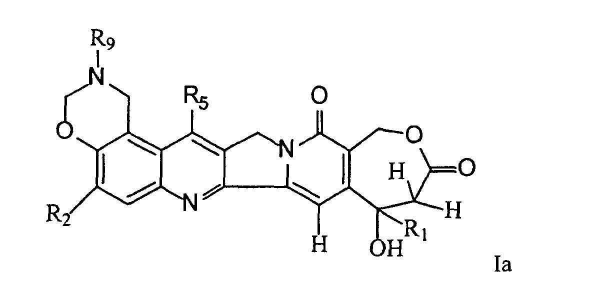 is acylated with a derivative of the -C(O)-A₁-NH₂ radical as defined in claim 1, in order to produce the β-hydroxylactonic compound of general formula I with Rₚ different from H.

## Patentansprüche

1. Eine Verbindung, **dadurch gekennzeichnet, daß** diese Verbindung der Formel (I) ist: 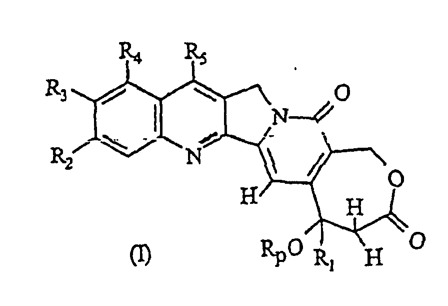 in racemischer Form, Enantiomerform oder allen Kombinationen dieser Formen, in der
R₁ eine Ethylgruppe darstellt;
R₂ H oder Halogen darstellt;
R₃ H, Niederalkyl, Halogen oder OR₆ darstellt, worin R₆ H, Niederalkyl oder Arylniederalkyl darstellt;
R₄ H oder -(CH₂)ₘNR₆R₇ darstellt, worin R₆ und R₇ unabhängig voneinander H oder Niederalkyl darstellen;
R₅ H, Niederalkyl oder -(CH₂)ₙ[N≈X], substituiert oder nicht substituiert, darstellt und [N≈X] eine Piperidyl-, Morpholinyl-, Piperazinyl- oder Indolyl-Gruppe darstellt und der Substituent Niederalkyl ist;
oder R₃ und R₄ einen Oxazin-Ring bilden, der ggf. an dem Stickstoffatom durch einen Rest R₉ substituiert ist, der Niederalkyl darstellt;
· Rₚ H oder eine leicht abspaltbare Gruppe der Formel -C(O)-(A₁)-NH₂ darstellt, in der A₁ (CH₂)ₘ oder einen verzweigten Niederalkylrest darstellt;
m eine ganze Zahl zwischen 0 und 6 ist;
n 1 oder 2 ist;
wobei, wenn Rₚ ein Wasserstoffatom ist, R₃ und R₄ zusammen einen ggf. substituierten Oxazin-Ring bilden,
und, wenn der Ausdruck "Nieder" sich auf Alkyl oder Alkylen bezieht, dieser höchstens 6 Kohlenstoffatome bedeutet;
oder ein pharmazeutisch verträgliches Salz davon.

2. Eine Verbindung nach Anspruch 1, **dadurch gekennzeichnet, daß** R₃ und R₄ einen ggf. substituierten Oxazin-Ring bilden; oder ein pharmazeutisch verträgliches Salz davon.

3. Eine Verbindung nach Anspruch 1, **dadurch gekennzeichnet, daß** Rₚ eine leicht abspaltbare Gruppe ist; oder ein pharmazeutisch verträgliches Salz davon.

4. Eine Verbindung nach Anspruch 2, **dadurch gekennzeichnet, daß** diese Verbindung aus den Verbindungen ausgewählt ist, die den folgenden Formeln entsprechen:
- 1,8-Diethyl-8,9-dihydro-8-hydroxy-2H,10H,12*H*-[1,3]oxazino[5,6-*f*]oxepino[3',4':6,7]indolizino[1,2-*b*] chinolin-10,13(15H)-dion;
- 8-Ethyl-8,9-dihydro-8-hydroxy-1-methyl-2*H*,10*H*,12*H*-[1,3]oxazino[5,6-*f*]oxepino[3',4':6,7]indolizino[1,2-*b*]-chinolin-10,13(15H)-dion;
- 8-Ethyl-8,9-dihydro-8-hydroxy-1-benzyl-2*H*,10*H*,12*H*-[1,3]oxazino[5,6-*f*]oxepino[3',4':6,7]indolizino[1,2-*b*]-chinolin-10,13(15*H*)-dion;
- 8-Ethyl-8,9-dihydro-4-fluor-8-hydroxy-1-benzyl-2*H*,10*H*,12*H*-[1,3]oxazino[5,6-*f*]oxepino[3',4':6.7]-indolizino[1,2-*b*]chinolin-10,13(15*H*)-dion;
oder ein pharmazeutisch verträgliches Salz davon.

5. Eine Verbindung nach Anspruch 3, **dadurch gekennzeichnet, daß** diese Verbindung aus den Verbindungen ausgewählt ist, die den folgenden Formeln entsprechen:
- 5-Ethyl-9,10-difluor-4,5-dihydro-5-(2-amino-1-oxoethoxy)-1*H*-oxepino[3',4':6,7]indolizino[1,2-*b*]chinolin-3,15(4*H*,13*H*)-dion;
- 5-Ethyl-9,10-difluor-4,5-dihydro-5-(2-amino-1-oxopropoxy)-1*H*-oxepino[3',4':6,7]indolizino[1,2-*b*]-chinolin-3,15(4*H*,13*H*)-dion; oder ein pharmazeutisch verträgliches Salz davon.

6. Eine Verbindung nach einem der vorhergehenden Ansprüche oder ein pharmazeutisch verträgliches Salz davon in Form eines Medikaments.

7. Pharmazeutische Zusammensetzung, die als Wirkstoff mindestens eine der Verbindungen nach einem der Ansprüche 1 bis 5 enthält.

8. Verwendung einer Verbindung nach einem der Ansprüche 1 bis 5 für die Herstellung von antitumoralen Medikamenten.

9. Verfahren zur Herstellung von Verbindungen der Formel Ia, die den Produkten der Formel I entsprechen, in der R₃ und R₄ einen Oxazin-Ring bilden, nach einem der Ansprüche 1, 2 oder 4, **dadurch gekennzeichnet, daß**;
- man eine β-Hydroxylacton-Verbindung der allgemeinen Formel D 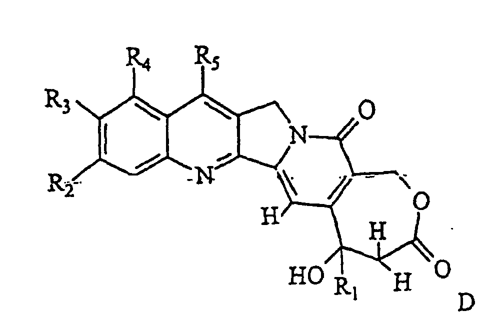
in der R₃ ein Hydroxyl-Rest ist, R₄ H ist, und R₁ und R₂ die oben angegebene Bedeutung haben, mit einem primären Amin unter Mannich-Bedingungen behandelt, um eine β-Hydroxylacton-Verbindung der allgemeinen Formel Ia zu erhalten, 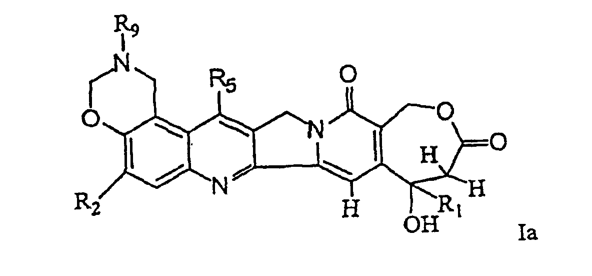 in der R₁, R₂, R₅ und R₉ die oben angegebene Bedeutung haben.

10. Verfahren zur Herstellung von Verbindungen der Formel Ib, die den Produkten der Formel I entsprechen, in der Rₚ nicht ein Wasserstoffatom ist, nach einem der Ansprüche 1, 2, 3 oder 5, **dadurch gekennzeichnet, daß**:
- man die Verbindung der allgemeinen Formel D 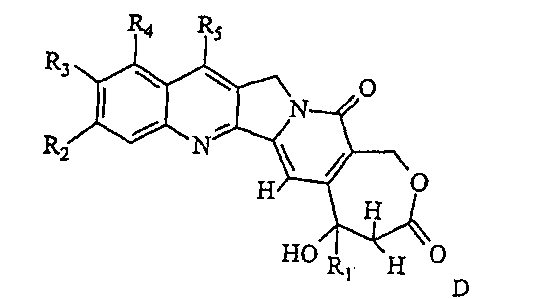
oder Ia 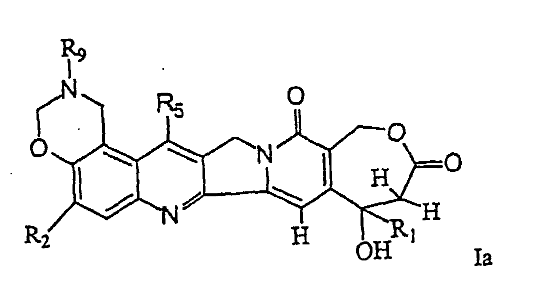 mit einem Derivat des Rests -C(O)-A₁-NH₂, wie in Anspruch 1 definiert, acyliert, um die β-Hydroxylacton-Verbindung der allgemeinen Formel I zu erhalten, worin Rₚ von H verschieden ist.
